# EUROPEAN PATENT SPECIFICATION

(11) **EP 3 749 673 B1**
(45) Date of publication and mention of the grant of the patent: **16.03.2022**
(21) Application number: 19701843.5
(22) Date of filing: 25.01.2019
(51) Int. Cl.: C07D 498/22, A61K 31/537, A61P 31/18

(54) **CRYSTALLINE FORM OF BICTEGRAVIR SODIUM**
KRISTALLINE FORM VON BICTEGRAVIR SODIUM
FORM CRISTALLINE DE SODIUM DE BICTEGRAVIR

(30) Priority: 09.02.2018 EP 18156094; 13.09.2018 EP 18194234
(43) Date of publication of application: 16.12.2020
(73) Proprietor: Sandoz AG, 4056 Basel (CH)
(72) Inventor: LENGAUER, Hannes, 6250 Kundl (AT); PICHLER, Arthur, 6250 Kundl (AT); MARGREITER, Renate, 6250 Kundl (AT)
(74) Representative: Kluschanzoff, Harald
(86) International application number: PCT/EP2019/051818
(87) International publication number: WO 2019/154634

(56) References cited:
- WO-A1-2015/196116
- US-A1- 2015 366 872
- CAIRA ED - MONTCHAMP JEAN-LUC: "Crystalline Polymorphism of Organic Compounds", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP008166276, ISSN: 0340-1022

## Description

### FIELD OF THE INVENTION

The present invention relates to a crystalline form of bictegravir sodium and to a process for its preparation. Furthermore, the invention relates to a pharmaceutical composition comprising said crystalline form of bictegravir sodium, preferably in a predetermined and/or effective amount, at least one pharmaceutically acceptable excipient and optionally one ore more additional antiviral agent(s). The pharmaceutical composition of the present invention can be used as a medicament, in particular for the treatment and/or prophylaxis of HIV-1 infections.

### BACKGROUND OF THE INVENTION

Bictegravir is a human immunodeficiency virus type 1 (HIV-1) integrase strand transfer inhibitor (INSTI). It is indicated for the treatment of HIV-1 infection in combination with nucleoside reverse transcriptase inhibitors (NRTIs) tenofovir alafenamide and emtricitabine. Bictegravir can be chemically designated as (2R,5S,13aR)-7,9-dioxo-10-((2,4,6-trifluorobenzyl)carbamoyl)-2,3,4,5,7,9,13,13a-octahydro-2,5-methanopyrido [1',2':4,5]pyrazino[2,1-b][1,3]oxazepin-8-olate (IUPAC name) or (2R,5S,13aR)-8-hydroxy-7,9-dioxo-*N*-(2,4,6-trifluorobenzyl)-2,3,4,5,7,9,13,13a-octahydro-2,5-methanopyrido [1',2':4,5]pyrazino[2,1-b][1,3]oxazepine-10 carboxamide and is represented by the following chemical structure according to Formula (I)

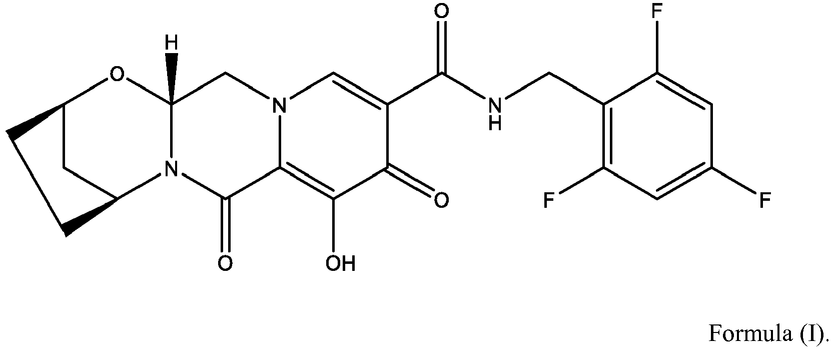

A process for the preparation of bictegravir is disclosed in Example 42 of WO 2014/100323 A1. WO 2015/196116 A1 discloses a crystalline form of bictegravir sodium designated as "Form I". Although the application mentions that the use of certain solvents, for example methanol, ethanol, water, isopropyl acetate, acetonitrile, tetrahydrofuran, methylisobutyl ketone and any mixture thereof produces different polymorphs, Form I is the only crystalline form disclosed therein.

Different solid state forms of an active pharmaceutical ingredient often possess different properties. Differences in the physicochemical properties of solid state forms can be important for the improvement of pharmaceutical compositions, for example, pharmaceutical formulations with improved dissolution profile or with improved stability or shelf-life can become accessible due to an improved solid state form of an active pharmaceutical ingredient. Also processing or handling of the active pharmaceutical ingredient during the formulation process may be improved. New solid state forms of an active pharmaceutical ingredient can thus have desirable processing properties. They can be easier to handle, better suited for storage, and/or allow for better purification, compared to previously known solid state forms.

The thermodynamically most stable polymorph of an active pharmaceutical ingredient is often used for the preparation of a drug product, since phase transitions during pharmaceutical standard processes like compaction, milling and wet granulation can be reduced to the extent possible. However, a huge drawback connected with the thermodynamically most stable form of a drug substance is the fact, that it is the least soluble form, which translates into decreased bioavailability. This is especially critical for low soluble compounds like bictegravir. Therefore, using a metastable polymorph of a drug substance may be desirable on account of its special properties, in particular higher solubility and hence bioavailability, but also better behavior during grinding and compression or lower hygroscopicity. However, it is important to avoid any possible solid form transitions, which may occour for a metastable polymorph during pharmaceutical processing or storage, since this can have a huge impact on the safety and efficacy of a drug product. Therefore, not every metastable polymorph of an active pharmaceutical ingredient can be used for the production of a pharmaceutical composition.

The objective of the present invention is to provide an improved polymorph of bictegravir sodium, which is kinetically stable e.g. does not undergo phase transformations during standard pharmaceutical processing and storage and at the same time shows high solubility and dissolution rates.

### SUMMARY OF THE INVENTION

The present invention solves the above mentioned problem by providing a metastable polymorph of bictegravir sodium, hereinafter also referred to "Form II". Form II of the present invention was found to be monotropically related to the known Form I of WO 2015/196116 A1, with the latter being the higher melting form. The transition temperature at which Form II of the present invention transforms to Form I of WO 2015/196116 A1 was found to be well above 300 °C, when measured with DSC at a heating rate of 10 K/min. This extraordinary high transition temperature reflects the high kinetic stability of Form II of the present invention and indicates that the probability of solid form changes during standard pharmaceutical processing and during storage is vanishingly small. On the other hand Form II possesses a higher solubility compared to Form I. Hence, bictegravir sodium Form II of the present invention combines the advantageous properties of high physical stability and high solubility and is therefore the preferred solid form of bictegravir sodium for the preparation of a pharmaceutical composition. Further advantageous properties of bictegravir sodium Form II are low hygroscopicity and a good behavior during grinding and compression, which render it particularly suitable for the production of a pharmaceutical composition.

**Abbreviations**

| | | |
|---|---|---|
| PXRD | powder X-ray diffractogramFTIR | Fourier transform infrared |
| ATR | attenuated total reflection | |
| DSC | differential scanning calorimetry | |
| TGA | thermogravimetric analysis | |

### Definitions

The term "bictegravir" as used herein refers to (2R,5S,13aR)-7,9-dioxo-10-((2,4,6-trifluorobenzyl)carbamoyl)-2,3,4,5,7,9,13,13a-octahydro-2,5-methanopyrido[1,2':4,5] pyrazino[2,1-b][1,3]oxazepin-8-olate (IUPAC name) or (2R,5S,13aR)-8-hydroxy-7,9-dioxo-*N*-(2,4,6-trifluorobenzyl)-2,3,4,5,7,9,13,13a-octahydro-2,5-methanopyrido[1',2':4,5]pyrazino [2,1-b][1,3]oxazepine-10 carboxamide according to Formula (I) disclosed herein above.

The term "bictegravir sodium" as used herein refers to the sodium salt of bictegravir, having a chemical structure, wherein about one mol of bictegravir is associated with one mol of sodium via ionic interaction of deprotonated bictegravir with Na⁺. Bictegravir sodium can be represented by the chemical structure according to Formula (II) hereinafter.

The term "bictegravir sodium Form I" as used herein, refers to the crystalline form of bictegravir sodium, which is disclosed in WO 2015/196116 A. Form I of bictegravir sodium can be characterized by having a powder X-ray diffractogram comprising reflections at 2-Theta angles of (5.5 ± 0.3)°, (16.1 ± 0.3)°, (22.1 ± 0.3)°, (23.3 ± 0.3)° and (28.5 ± 0.3)°, when measured at a temperature in the range of from 20 to 30 °C with Cu-Kalpha₁,₂ radiation having a wavelength of 0.15419 nm.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% relative humidity, preferably 30-70% relative humidity, more preferably 40-60% relative humidity and most preferably 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials " by Vitalij K. Pecharsky and Peter Y. Zavalij, Kluwer Academic Publishers, 2003, page 3).

As used herein, the term "amorphous" refers to a solid form of a compound that is not crystalline. An amorphous compound possesses no long-range order and does not display a definitive X-ray diffraction pattern with reflections.

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 6.5° 2-Theta for example can appear between 6.3° and 6.7° 2-Theta, preferably between 6.4 and 6.6° 2-Theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "essentially the same" with reference to Fourier transform infrared spectroscopy means that variabilities in peak positions and relative intensities of the peaks are to be taken into account. For example, a typical precision of the wavenumber values is in the range of ± 2 cm⁻¹. Thus, a peak at 3055 cm⁻¹ for example can appear in the range of from 3053 to 3057 cm⁻¹ on most infrared spectrometers under standard conditions. Differences in relative intensities are typically smaller compared to X-ray diffraction. However, one skilled in the art will appreciate that small differences in peak intensities due to degree of crystallinity, sample preparation and other factors can also occur in infrared spectroscopy. Relative peak intensities should therefore be taken as qualitative measure only.

The term "essentially the same" with reference to Raman spectroscopy means that variabilities in peak positions and relative intensities of the peaks are to be taken into account. For example, a typical precision of the wavenumber values is in the range of ± 4 cm⁻¹. Thus, a peak at 1641 cm⁻¹ for example can appear in the range of from 1637 to 1645 cm⁻¹ on most Raman spectrometers under standard conditions. Differences in relative intensities are typically smaller compared to X-ray diffraction. However, one skilled in the art will appreciate that small differences in peak intensities due to degree of crystallinity, sample preparation and other factors can also occur in Raman spectroscopy. Relative peak intensities should therefore be taken as qualitative measure only.

As used herein, the term "substantially free of any other physical form" with reference to a composition comprising a particular physical form of bictegravir sodium means that the composition includes at most 20%, preferably at most 10%, more preferably at most 5%, even more preferably at most 2% and most preferably at most 1% by weight of any other physical form of bictegravir sodium, based on the weight of the composition.

The term "physical form" as used herein refers to any crystalline and/or amorphous phase of a compound.

As used herein the term"solvate" refers to a crystalline form of a molecule, atom, and/or ion that further comprises molecules of a solvent or solvents incorporated into the crystalline lattice structure.

Crystalline forms of bictegravir sodium may be referred to herein as being characterized by a powder X-ray diffractogram, a FTIR spectrum or a Raman spectrum "as shown in" a figure. The person skilled in the art understands that factors such as variations in instrument type, response and variations in sample directionality, sample concentration, sample purity, sample history and sample preparation may lead to variations, for example relating to the exact reflection and peak positions and their intensities. However, a comparison of the graphical data in the figures herein with the graphical data generated for an unknown physical form and the confirmation that two sets of graphical data relate to the same crystal form is well within the knowledge of a person skilled in the art.

As used herein, the term "mother liquor" refers to the solution remaining after crystallization of a solid from said solution.

A "predetermined amount" as used herein with regard to bictegravir sodium refers to the initial amount of bictegravir sodium used for the preparation of a pharmaceutical composition having a desired dosage strength of bictegravir.

The term "effective amount" as used herein with regard to bictegravir sodium encompasses an amount of bictegravir sodium, which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient.

### BRIEF DESCRIPTION OF THE FIGURES

**Figure 1****:** illustrates a representative PXRD of bictegravir sodium Form II of the present invention. The x-axis shows the scattering angle in °2-Theta, the y-axis shows the intensity of the scattered X-ray beam in counts of detected photons.
**Figure 2****:** illustrates a representative PXRD of bictegravir sodium 2,2,2-trifluoroethanol solvate of the present invention. The x-axis shows the scattering angle in °2-Theta, the y-axis shows the intensity of the scattered X-ray beam in counts of detected photons.
**Figure 3**: illustrates a comparison of a representative PXRD of crystalline Form II of bictegravir sodium of the present invention (bottom) and a representative PXRD of Form I of bictegravir sodium of WO 2015/196116 A1 prepared according to Reference Example 1 herein (top). The x-axis shows the scattering angle in °2-Theta. The PXRD of Form I was shifted along the y-axis to separate the diffractograms for clarity.

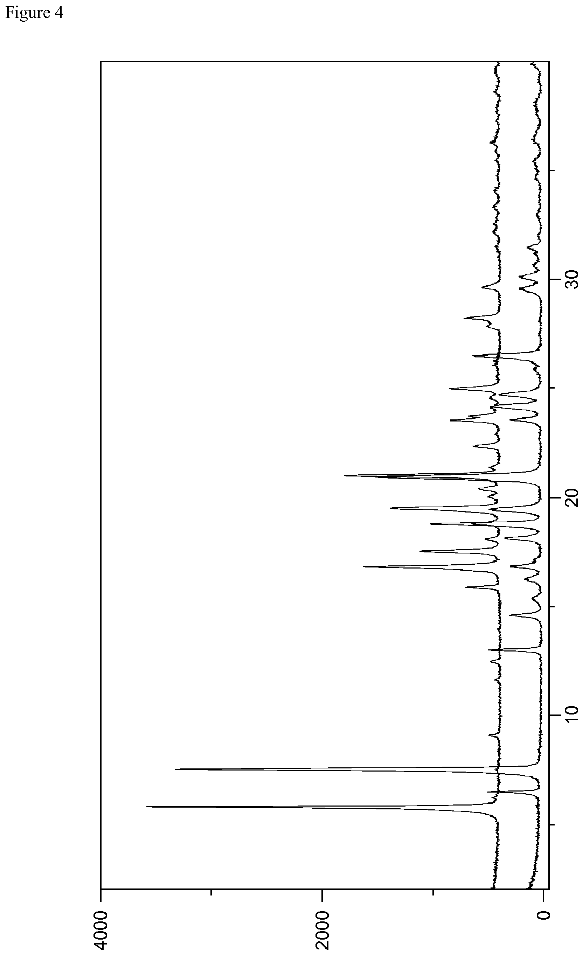

**Figure 4****:** illustrates a comparison of a representative PXRD of crystalline Form II of bictegravir sodium of the present invention (bottom) and a representative PXRD of bictegravir sodium 2,2,2-trifluoroethanol solvate of the present invention (top). The x-axis shows the scattering angle in °2-Theta. The PXRD of the 2,2,2-trifluoroethanol solvate was shifted along the y-axis to separate the diffractograms for clarity.
**Figure 5****:** illustrates a representative FTIR spectrum of bictegravir sodium Form II of the present invention. The x-axis shows the wavenumbers in cm⁻¹, the y-axis shows the relative intensity in percent transmittance.
**Figure 6****:** illustrates a representative FTIR spectrum of bictegravir sodium 2,2,2-trifluoroethanol solvate of the present invention. The x-axis shows the wavenumbers in cm⁻¹, the y-axis shows the relative intensity in percent transmittance.
**Figure 7****:** illustrates a representative Raman spectrum of bictegravir sodium Form II of the present invention. The x-axis shows the wavenumbers in cm⁻¹, the y-axis shows the Raman intensity.
**Figure 8****:** illustrates a representative Raman spectrum of bictegravir sodium 2,2,2-trifluoroethanol solvate of the present invention. The x-axis shows the wavenumbers in cm⁻¹, the y-axis shows the Raman intensity.
**Figure 9****:** illustrates a representative DSC curve of bictegravir sodium Form II of the present invention. The x-axis shows the temperature in degree Celsius (°C), the y-axis shows the heat flow rate in Watt per gram (W/g) with endoothermic peaks going up.
**Figure 10****:** illustrates a representative DSC curve of bictegravir sodium 2,2,2-trifluoroethanol solvate of the present invention. The x-axis shows the temperature in degree Celsius (°C), the y-axis shows the heat flow rate in Watt per gram (W/g) with endothermic peaks going up.
**Figure 11****:** illustrates a representative TGA curve of bictegravir sodium Form II of the present invention. The x-axis shows the temperature in degree Celsius (°C), the y-axis shows the mass (loss) of the sample in weight percent (weight%).
**Figure 12****:** illustrates a representative TGA curve of bictegravir sodium 2,2,2-trifluoroethanol solvate of the present invention. The x-axis shows the temperature in degree Celsius (°C), the y-axis shows the mass (loss) of the sample in weight percent (weight%).

### DETAILED DESCRIPTION OF THE INVENTION

The present invention provides a metastable polymorph of bictegravir sodium, herein also designated as "Form II".

Despite being a metastable polymorph, Form II of bictegravir sodium of the present invention possesses high physical stability, e.g. it only transforms to the themodynamically more stable Form I of WO 2015/196116 A1 at temperatures well above 300 °C, when measured with DSC at a heating rate of 10 K/min. Form II of the present invention shows higher solubility, a fact which translates in higher bioequivalency of a low soluble drug like bictegravir. Hence, Form II of the present invention combines the advantageous properties of high physical stability and high solubility and is therefore the ideal solid form of bictegravir sodium for the preparation of an improved pharmaceutical composition e.g. a pharmaceutical composition with increased bioavailability, which is safe and effective during its whole shelf-life.

Bictegravir sodium can be represented by the following chemical structure according to Formula (II)

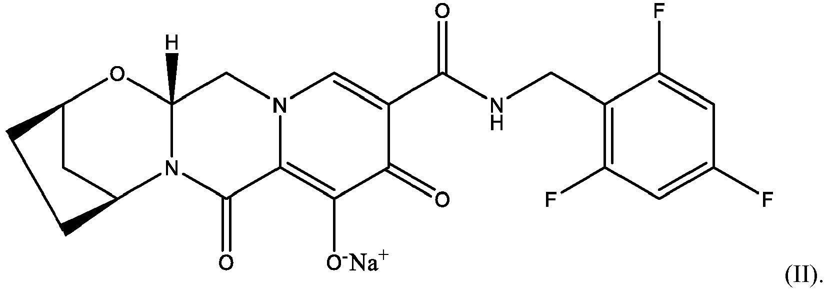

Bictegravir sodium can be characterized by a molar ratio of bictegravir and sodium preferably in the range of from 1.0 : 0.7 to 1.0 : 1.3, more preferably in the range of from 1.0 : 0.8 to 1.0 : 1.2, even more preferably in the range of from 1.0 : 0.9 to 1.0 : 1.1 and in particular the molar ratio is 1.0 : 1.0.

Bictegravir sodium Form II of the present invention may be characterized by any one of the following embodiments or by combining two or more of the following embodiments.

Hence, in a first aspect the present invention relates to a crystalline form of bictegravir sodium (Form II) that can be characterized by having a powder X-ray diffractogram comprising reflections at 2-Theta angles of:
(6.5 ± 0.2)°, (7.5 ± 0.2)° and (18.8 ± 0.2)°; or
(6.5 ± 0.2)°, (7.5 ± 0.2)°, (18.8 ± 0.2)° and (20.9 ± 0.2)°; or
(6.5 ± 0.2)°, (7.5 ± 0.2)°, (18.8 ± 0.2)°, (19.4 ± 0.2)° and (20.9 ± 0.2)°; or
(6.5 ± 0.2)°, (7.5 ± 0.2)°, (13.0 ± 0.2)°, (18.8 ± 0.2)°, (19.4 ± 0.2)° and (20.9 ± 0.2)°; or
(6.5 ± 0.2)°, (7.5 ± 0.2)°, (13.0 ± 0.2)°, (18.8 ± 0.2)°, (19.4 ± 0.2)°, (20.9 ± 0.2)° and (26.5 ± 0.2)°; or
(6.5 ± 0.2)°, (7.5 ± 0.2)°, (13.0 ± 0.2)°, (18.1 ± 0.2)°, (18.8 ± 0.2)°, (19.4 ± 0.2)°, (20.9 ± 0.2)° and (26.5 ± 0.2)°; or
(6.5 ± 0.2)°, (7.5 ± 0.2)°, (13.0 ± 0.2)°, (16.8 ± 0.2)°, (18.1 ± 0.2)°, (18.8 ± 0.2)°, (19.4 ± 0.2)°, (20.9 ± 0.2)° and (26.5 ± 0.2)°; or
(6.5 ± 0.2)°, (7.5 ± 0.2)°, (13.0 ± 0.2)°, (14.6 ± 0.2)°, (16.8 ± 0.2)°, (18.1 ± 0.2)°, (18.8 ± 0.2)°, (19.4 ± 0.2)°, (20.9 ± 0.2)° and (26.5 ± 0.2)°; or
(6.5 ± 0.2)°, (7.5 ± 0.2)°, (13.0 ± 0.2)°, (14.6 ± 0.2)°, (16.8 ± 0.2)°, (18.1 ± 0.2)°, (18.8 ± 0.2)°, (19.4 ± 0.2)°, (20.9 ± 0.2)°, (24.2 ± 0.2)° and (26.5 ± 0.2)°; or
(6.5 ± 0.2)°, (7.5 ± 0.2)°, (13.0 ± 0.2)°, (14.6 ± 0.2)°, (16.8 ± 0.2)°, (18.1 ± 0.2)°, (18.8 ± 0.2)°, (19.4 ± 0.2)°, (20.9 ± 0.2)°, (24.2 ± 0.2)°, (24.7 ± 0.2)° and (26.5 ± 0.2)°; or
(6.5 ± 0.2)°, (7.5 ± 0.2)°, (13.0 ± 0.2)°, (14.6 ± 0.2)°, (16.8 ± 0.2)°, (18.1 ± 0.2)°, (18.8 ± 0.2)°, (19.4 ± 0.2)°, (20.9 ± 0.2)°, (23.5 ± 0.2)°, (24.2 ± 0.2)°, (24.7 ± 0.2)° and (26.5 ± 0.2)°,
when measured at a temperature in the range of from 20 to 30 °C with Cu-Kalpha₁,₂ radiation having a wavelength of 0.15419 nm.

In another embodiment the present invention relates to a crystalline form of bictegravir sodium (Form II) that can be characterized by having a powder X-ray diffractogram comprising reflections at 2-Theta angles of:
(6.5 ± 0.1)°, (7.5 ± 0.1)° and (18.8 ± 0.1)°; or
(6.5 ± 0.1)°, (7.5 ± 0.1)°, (18.8 ± 0.1)° and (20.9 ± 0.1)°; or
(6.5 ± 0.1)°, (7.5 ± 0.1)°, (18.8 ± 0.1)°, (19.4 ± 0.1)° and (20.9 ± 0.1)°; or
(6.5 ± 0.1)°, (7.5 ± 0.1)°, (13.0 ± 0.1)°, (18.8 ± 0.1)°, (19.4 ± 0.1)° and (20.9 ± 0.1)°; or
(6.5 ± 0.1)°, (7.5 ± 0.1)°, (13.0 ± 0.1)°, (18.8 ± 0.1)°, (19.4 ± 0.1)°, (20.9 ± 0.1)° and (26.5 ± 0.1)°; or
(6.5 ± 0.1)°, (7.5 ± 0.1)°, (13.0 ± 0.1)°, (18.1 ± 0.1)°, (18.8 ± 0.1)°, (19.4 ± 0.1)°, (20.9 ± 0.1)° and (26.5 ± 0.1)°; or
(6.5 ± 0.1)°, (7.5 ± 0.1)°, (13.0 ± 0.1)°, (16.8 ± 0.1)°, (18.1 ± 0.1)°, (18.8 ± 0.1)°, (19.4 ± 0.1)°, (20.9 ± 0.1)° and (26.5 ± 0.1)°; or
(6.5 ± 0.1)°, (7.5 ± 0.1)°, (13.0 ± 0.1)°, (14.6 ± 0.1)°, (16.8 ± 0.1)°, (18.1 ± 0.1)°, (18.8 ± 0.1)°, (19.4 ± 0.1)°, (20.9 ± 0.1)° and (26.5 ± 0.1)°; or
(6.5 ± 0.1)°, (7.5 ± 0.1)°, (13.0 ± 0.1)°, (14.6 ± 0.1)°, (16.8 ± 0.1)°, (18.1 ± 0.1)°, (18.8 ± 0.1)°, (19.4 ± 0.1)°, (20.9 ± 0.1)°, (24.2 ± 0.1)° and (26.5 ± 0.1)°; or
(6.5 ± 0.1)°, (7.5 ± 0.1)°, (13.0 ± 0.1)°, (14.6 ± 0.1)°, (16.8 ± 0.1)°, (18.1 ± 0.1)°, (18.8 ± 0.1)°, (19.4 ± 0.1)°, (20.9 ± 0.1)°, (24.2 ± 0.1)°, (24.7 ± 0.1)° and (26.5 ± 0.1)°; or
(6.5 ± 0.1)°, (7.5 ± 0.1)°, (13.0 ± 0.1)°, (14.6 ± 0.1)°, (16.8 ± 0.1)°, (18.1 ± 0.1)°, (18.8 ± 0.1)°, (19.4 ± 0.1)°, (20.9 ± 0.1)°, (23.5 ± 0.1)°, (24.2 ± 0.1)°, (24.7 ± 0.1)° and (26.5 ± 0.1)°,
when measured at a temperature in the range of from 20 to 30 °C with Cu-Kalpha₁,₂ radiation having a wavelength of 0.15419 nm.

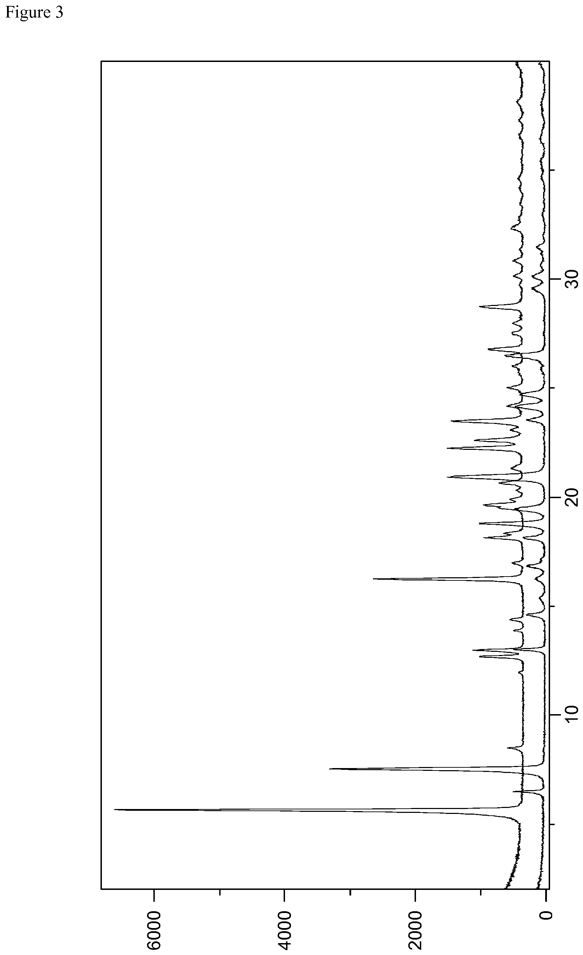

The powder X-ray diffractogram of bictegravir sodium Form II of the present invention is readily distinguishable from the known Form I of WO 2015/196116 A1 (see also the overlay displayed in Figure 3 herein for this purpose). Form II shows for example reflections at 2-Theta angles of (6.5 ± 0.2)°, (7.5 ± 0.2)° and (18.8 ± 0.2)°, whereas Form I shows no reflection in these ranges. On the other hand, Form II shows no reflection at (5.5 ± 0.2)° 2-Theta, whereas Form I possesses a very characteristic reflection (namely the most intensive reflection) in this range. Thus, in another aspect, the present invention relates to a crystalline form of bictegravir sodium (Form II) which can be characterized by having a powder X-ray diffractogram comprising reflections at 2-Theta angles as described above, but comprising no reflections in the range of (5.5 ± 0.2)° 2-Theta, when measured at a temperature in the range of from 20 to 30 °C with Cu-Kalpha_{1,2} radiation having a wavelength of 0.15419 nm.

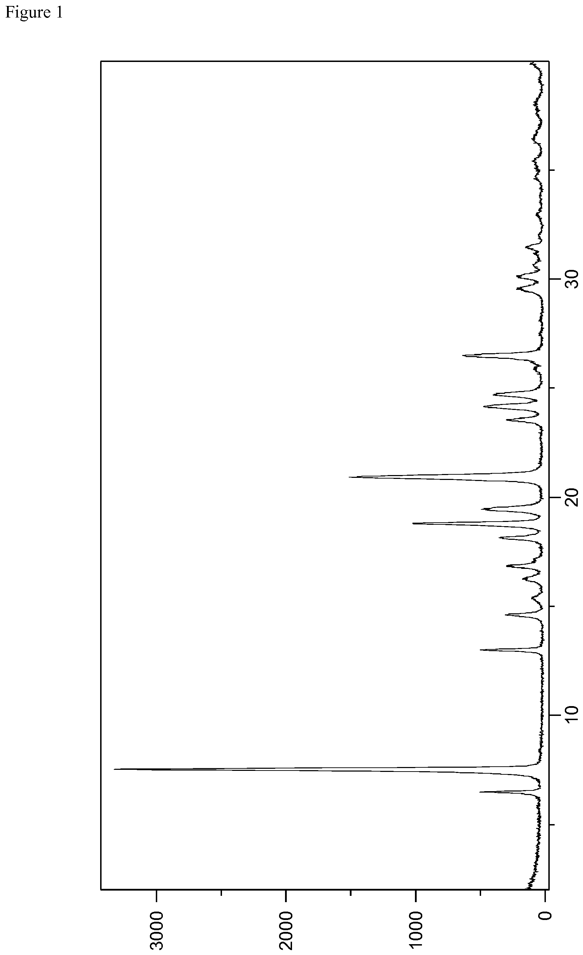

In yet another embodiment, the present invention relates to a crystalline form of bictegravir sodium (Form II) characterized by having a powder X-ray diffractogram essentially the same as shown in Figure 1 of the present invention, when measured at a temperature in the range of from 20 to 30 °C with Cu-Kalpha_{1,2} radiation having a wavelength of 0.15419 nm.

In a further embodiment, the present invention relates to a crystalline form of bictegravir sodium (Form II) characterized by having a FTIR spectrum comprising peaks at wavenumbers of:
(3055 ± 2) cm⁻¹, (2964 ± 2) cm⁻¹ and (1614 ± 2) cm⁻¹ or;
(3055 ± 2) cm⁻¹, (2964 ± 2) cm⁻¹, (1614 ± 2) cm⁻¹ and (1525 ± 2) cm-¹; or
(3055 ± 2) cm⁻¹, (2964 ± 2) cm⁻¹, (1614 ± 2) cm⁻¹, (1525 ± 2) cm⁻¹ and (1435 ± 2) cm⁻¹; or
(3055 ± 2) cm⁻¹, (2964 ± 2) cm⁻¹, (1614 ± 2) cm⁻¹, (1525 ± 2) cm⁻¹, (1435 ± 2) cm⁻¹ and (1315 ± 2) cm⁻¹; or
(3055 ± 2) cm⁻¹, (2964 ± 2) cm⁻¹, (1614 ± 2) cm⁻¹, (1525 ± 2) cm⁻¹, (1435 ± 2) cm⁻¹, (1315 ± 2) cm⁻¹ and (1290 ± 2) cm⁻¹; or
(3055 ± 2) cm⁻¹, (2964 ± 2) cm⁻¹, (1614 ± 2) cm⁻¹, (1525 ± 2) cm⁻¹, (1435 ± 2) cm⁻¹, (1315 ± 2) cm⁻¹, (1290 ± 2) cm⁻¹ and (1252 ± 2) cm⁻¹; or
(3055 ± 2) cm⁻¹, (2964 ± 2) cm⁻¹, (1614 ± 2) cm⁻¹, (1525 ± 2) cm⁻¹, (1435 ± 2) cm⁻¹, (1315 ± 2) cm⁻¹, (1290 ± 2) cm⁻¹, (1252 ± 2) cm⁻¹ and (1069 ± 2) cm-¹; or
(3055 ± 2) cm⁻¹, (2964 ± 2) cm⁻¹, (1614 ± 2) cm⁻¹, (1525 ± 2) cm⁻¹, (1435 ± 2) cm⁻¹, (1315 ± 2) cm⁻¹, (1290 ± 2) cm⁻¹, (1252 ± 2) cm⁻¹, (1069 ± 2) cm⁻¹ and (998 ± 2) cm⁻¹,
when measured at a temperature in the range of from 20 to 30 °C with a diamond ATR cell.

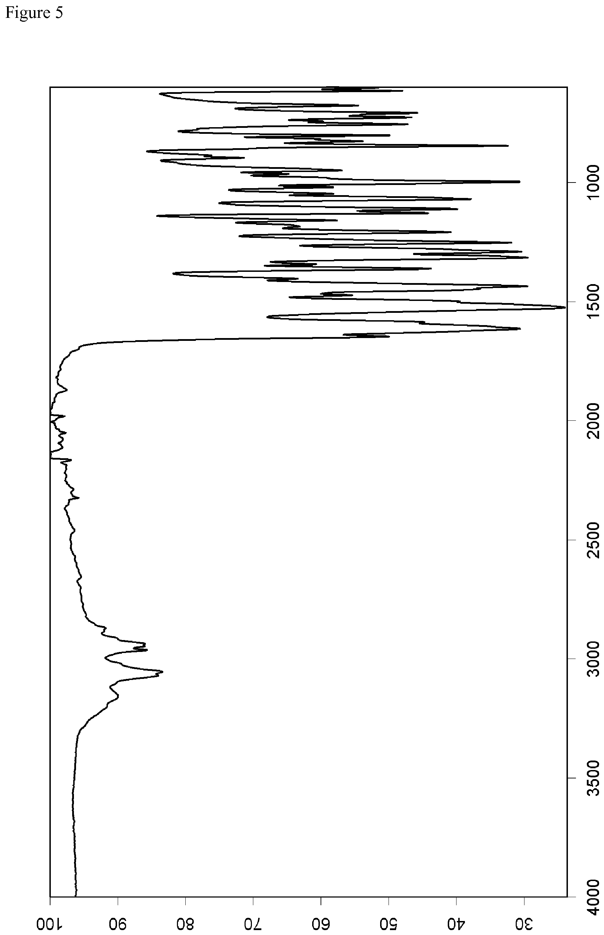

In yet another embodiment, the present invention relates to a crystalline form of bictegravir sodium (Form II) characterized by having a FTIR spectrum essentially the same as shown in Figure 5 of the present invention, when measured at a temperature in the range of from 20 to 30 °C with a diamond ATR cell.

In a further embodiment, the present invention relates to a crystalline form of bictegravir sodium (Form II) characterized by having a Raman spectrum comprising peaks at wavenumbers of:
(1641 ± 4) cm⁻¹, (1436 ± 4) cm⁻¹ and (680 ± 4) cm⁻¹; or
(1641 ± 4) cm⁻¹, (1436 ± 4) cm⁻¹, (1404 ± 4) cm⁻¹ and (680 ± 4) cm-¹; or
(1641 ± 4) cm⁻¹, (1436 ± 4) cm⁻¹, (1404 ± 4) cm⁻¹, (1328 ± 4) cm⁻¹ and (680 ± 4) cm⁻¹; or
(1641 ± 4) cm⁻¹, (1436 ± 4) cm⁻¹, (1404 ± 4) cm⁻¹, (1328 ± 4) cm⁻¹, (1254 ± 4) cm⁻¹ and (680 ± 4) cm⁻¹; or
(1641 ± 4) cm⁻¹, (1436 ± 4) cm⁻¹, (1404 ± 4) cm⁻¹, (1328 ± 4) cm⁻¹, (1254 ± 4) cm⁻¹, (759 ± 4) cm⁻¹ and (680 ± 4) cm⁻¹; or
(1641 ± 4) cm⁻¹, (1474 ± 4) cm⁻¹, (1436 ± 4) cm⁻¹, (1404 ± 4) cm⁻¹, (1328 ± 4) cm⁻¹, (1254 ± 4) cm⁻¹, (759 ± 4) cm⁻¹ and (680 ± 4) cm⁻¹; or
(1641 ± 4) cm⁻¹, (1517 ± 4) cm⁻¹, (1474 ± 4) cm⁻¹, (1436 ± 4) cm⁻¹, (1404 ± 4) cm⁻¹, (1328 ± 4) cm⁻¹, (1254 ± 4) cm⁻¹, (759 ± 4) cm⁻¹ and (680 ± 4) cm⁻¹; or
(1641 ± 4) cm⁻¹, (1586 ± 4) cm⁻¹, (1517 ± 4) cm⁻¹, (1474 ± 4) cm⁻¹, (1436 ± 4) cm⁻¹, (1404 ± 4) cm⁻¹, (1328 ± 4) cm⁻¹, (1254 ± 4) cm⁻¹, (759 ± 4) cm⁻¹ and (680 ± 4) cm⁻¹,
when measured at a temperature in the range of from 20 to 30 °C and a wavelength of 785 nm.

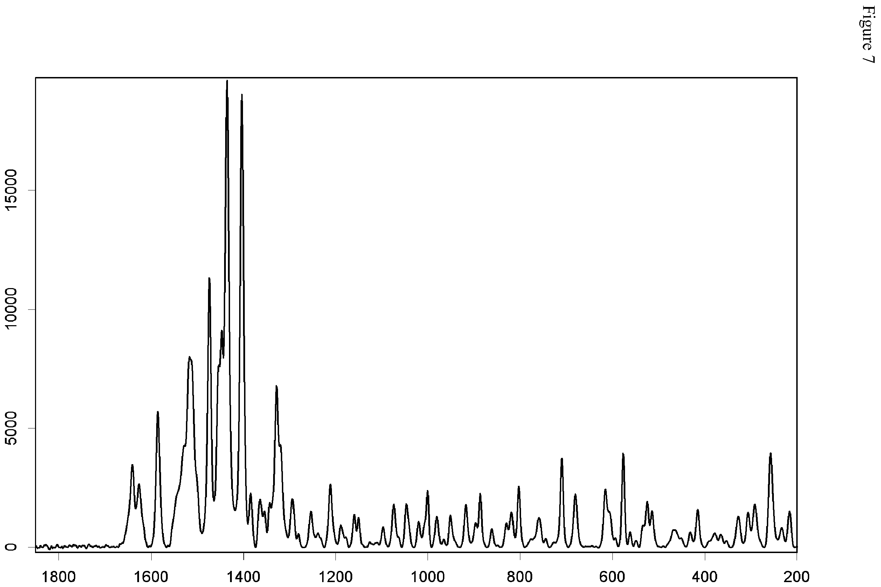

In yet another embodiment, the present invention relates to a crystalline form of bictegravir sodium (Form II) characterized by having a Raman spectrum essentially the same as shown in Figure 7 of the present invention, when measured at a temperature in the range of from 20 to 30 °C and a wavelength of 785 nm.

In one aspect, the present invention relates to a composition comprising Form II of bictegravir sodium of the present invention, which is essentially free of any other physical forms of bictegravir sodium. For example, a composition comprising Form II of bictegravir sodium of the present invention comprises at most 20 weight%, preferably at most 10 weight%, more preferably at most 5 weight%, even more preferably at most 2 weight% and most preferably at most 1 weight% of any other physical form of bictegravir sodium, based on the weight of the composition. Preferably, the any other physical form of bictegravir sodium is selected from the group consisting of Form I of WO 2015/196116 A1, the 2,2,2-trifluoroethanol solvate as defined hereinafter or amorphous bictegravir sodium. Hence, in a further preferred embodiment, the invention relates to a composition comprising bictegravir sodium Form II as defined herein above, characterized by having a PXRD comprising no reflections at 2-Theta angles in the range of (5.5 ± 0.2)° and/or (5.8 ± 0.2)°, when measured at a temperature in the range of from 20 to 30 °C with Cu-Kalpha_{1,2} radiation having a wavelength of 0.15419 nm.

It was surprisingly found that a novel crystalline form of bictegravir sodium can be obtained by crystallizing bictegravir sodium from 2,2,2-trifluoroethanol and applying the conditions as described below. The crystallization of bictegravir sodium from 2,2,2-trifluoroethanol under the hereinafter disclosed conditions leads to the formation of a 2,2,2-trifluoroethanol solvate, which can be further desolvated under the hereinafter disclosed drying conditions to the preferred bictegravir sodium form II of the present invention. Hence, the 2,2,2-trifluoroethanol solvate is an intermediate in the preparation of bictegravir sodium Form II of the present invention.

The bictegravir sodium 2,2,2-trifluoroethanol solvate of the present invention may be characterized by any one of the following embodiments or by combining two or more of the following embodiments.

In one aspect the present invention relates to the 2,2,2-trifluoroethanol solvate of bictegravir sodium which can be characterized by having a powder X-ray diffractogram comprising reflections at 2-Theta angles of:
(5.8 ± 0.2)°, (16.8 ± 0.2)° and (21.0 ± 0.2)°; or
(5.8 ± 0.2)°, (16.8 ± 0.2)°, (19.5 ± 0.2)° and (21.0 ± 0.2)°; or
(5.8 ± 0.2)°, (16.8 ± 0.2)°, (17.5 ± 0.2)°, (19.5 ± 0.2)° and (21.0 ± 0.2)°; or
(5.8 ± 0.2)°, (15.9 ± 0.2)°, (16.8 ± 0.2)°, (17.5 ± 0.2)°, (19.5 ± 0.2)° and (21.0 ± 0.2)°; or
(5.8 ± 0.2)°, (9.1 ± 0.2)°, (15.9 ± 0.2)°, (16.8 ± 0.2)°, (17.5 ± 0.2)°, (19.5 ± 0.2)° and (21.0 ± 0.2)°; or
(5.8 ± 0.2)°, (9.1 ± 0.2)°, (12.5 ± 0.2)°, (15.9 ± 0.2)°, (16.8 ± 0.2)°, (17.5 ± 0.2)°, (19.5 ± 0.2)° and (21.0 ± 0.2)°; or
(5.8 ± 0.2)°, (9.1 ± 0.2)°, (12.5 ± 0.2)°, (15.9 ± 0.2)°, (16.8 ± 0.2)°, (17.5 ± 0.2)°, (19.5 ± 0.2)°, (21.0 ± 0.2)° and (25.0 ± 0.2)°; or
(5.8 ± 0.2)°, (9.1 ± 0.2)°, (12.5 ± 0.2)°, (15.9 ± 0.2)°, (16.8 ± 0.2)°, (17.5 ± 0.2)°, (19.5 ± 0.2)°, (21.0 ± 0.2)°, (22.3 ± 0.2)° and (25.0 ± 0.2)°;
when measured at a temperature in the range of from 20 to 30 °C with Cu-Kalpha₁,₂ radiation having a wavelength of 0.15419 nm.

In another embodiment, the present invention relates to the 2,2,2-trifluoroethanol solvate of bictegravir sodium which can be characterized by having a powder X-ray diffractogram comprising reflections at 2-Theta angles of:
(5.8 ± 0.1)°, (16.8 ± 0.1)° and (21.0 ± 0.1)°; or
(5.8 ± 0.1)°, (16.8 ± 0.1)°, (19.5 ± 0.1)° and (21.0 ± 0.1)°; or
(5.8 ± 0.1)°, (16.8 ± 0.1)°, (17.5 ± 0.1)°, (19.5 ± 0.1)° and (21.0 ± 0.1)°; or
(5.8 ± 0.1)°, (15.9 ± 0.1)°, (16.8 ± 0.1)°, (17.5 ± 0.1)°, (19.5 ± 0.1)° and (21.0 ± 0.1)°; or
(5.8 ± 0.1)°, (9.1 ± 0.1)°, (15.9 ± 0.1)°, (16.8 ± 0.1)°, (17.5 ± 0.1)°, (19.5 ± 0.1)° and (21.0 ± 0.1)°; or
(5.8 ± 0.1)°, (9.1 ± 0.1)°, (12.5 ± 0.1)°, (15.9 ± 0.1)°, (16.8 ± 0.1)°, (17.5 ± 0.1)°, (19.5 ± 0.1)° and (21.0 ± 0.1)°; or
(5.8 ± 0.1)°, (9.1 ± 0.1)°, (12.5 ± 0.1)°, (15.9 ± 0.1)°, (16.8 ± 0.1)°, (17.5 ± 0.1)°, (19.5 ± 0.1)°, (21.0 ± 0.1)° and (25.0 ± 0.1)°; or
(5.8 ± 0.1)°, (9.1 ± 0.1)°, (12.5 ± 0.1)°, (15.9 ± 0.1)°, (16.8 ± 0.1)°, (17.5 ± 0.1)°, (19.5 ± 0.1)°, (21.0 ± 0.1)°, (22.3 ± 0.1)° and (25.0 ± 0.1)°;
when measured at a temperature in the range of from 20 to 30 °C with Cu-Kalpha₁,₂ radiation having a wavelength of 0.15419 nm.

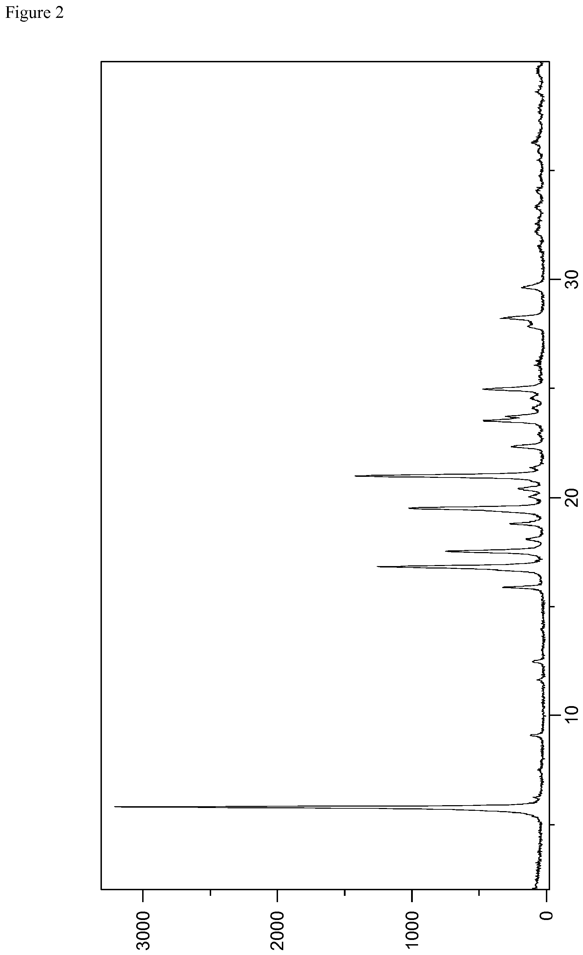

In yet another embodiment, the present invention relates to the 2,2,2-trifluoroethanol solvate of bictegravir sodium
characterized by having a powder X-ray diffractogram essentially the same as shown in Figure 2 of the present invention, when measured at a temperature in the range of from 20 to 30 °C with Cu-Kalpha_{1,2} radiation having a wavelength of 0.15419 nm.

In a further embodiment, the present invention relates to the 2,2,2-trifluoroethanol solvate of bictegravir characterized
by having a FTIR spectrum comprising peaks at wavenumbers of:
(3069 ± 2) cm⁻¹, (2933 ± 2) cm⁻¹ and (1526 ± 2) cm⁻¹ or;
(3069± 2) cm⁻¹, (2933 ± 2) cm⁻¹, (1526 ± 2) cm⁻¹ and (1290 ± 2) cm-¹; or
(3069± 2) cm⁻¹, (2933 ± 2) cm⁻¹, (1623 ± 2) cm⁻¹, (1526 ± 2) cm⁻¹ and (1290 ± 2) cm⁻¹; or
(3069± 2) cm⁻¹, (2933 ± 2) cm⁻¹, (1623 ± 2) cm⁻¹, (1526 ± 2) cm⁻¹, (1434 ± 2) cm⁻¹ and (1290 ± 2) cm⁻¹; or
(3069± 2) cm⁻¹, (2933 ± 2) cm⁻¹, (1623 ± 2) cm⁻¹, (1526 ± 2) cm⁻¹, (1434 ± 2) cm⁻¹, (1290 ± 2) cm⁻¹ and (1207 ± 2) cm⁻¹; or
(3069± 2) cm⁻¹, (2933 ± 2) cm⁻¹, (1623 ± 2) cm⁻¹, (1526 ± 2) cm⁻¹, (1434 ± 2) cm⁻¹, (1290 ± 2) cm⁻¹, (1207 ± 2) cm⁻¹ and (1073 ± 2) cm⁻¹; or
(3069± 2) cm⁻¹, (2933 ± 2) cm⁻¹, (1623 ± 2) cm⁻¹, (1526 ± 2) cm⁻¹, (1434 ± 2) cm⁻¹, (1290 ± 2) cm⁻¹, (1207 ± 2) cm⁻¹, (1073 ± 2) cm⁻¹ and (998 ± 2) cm⁻¹; or
(3069± 2) cm⁻¹, (2933 ± 2) cm⁻¹, (1623 ± 2) cm⁻¹, (1526 ± 2) cm⁻¹, (1434 ± 2) cm⁻¹, (1290 ± 2) cm⁻¹, (1207 ± 2) cm⁻¹, (1073 ± 2) cm⁻¹, (998 ± 2) cm⁻¹ and (832 ± 2) cm⁻¹,
when measured at a temperature in the range of from 20 to 30 °C with a diamond ATR cell.

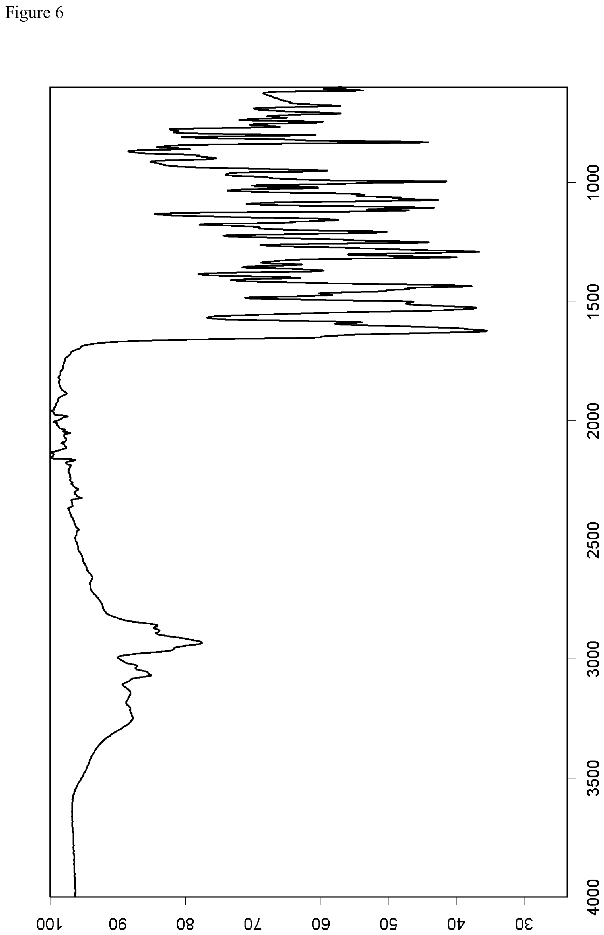

In yet another embodiment, the present invention relates to the 2,2,2-trifluoroethanol solvate of bictegravir sodium
characterized by having a FTIR spectrum essentially the same as shown in Figure 6 of the present invention, when measured at a temperature in the range of from 20 to 30 °C with a diamond ATR cell.

In a further embodiment, the present invention relates to the 2,2,2-trifluoroethanol solvate of bictegravir sodium
characterized by having a Raman spectrum comprising peaks at wavenumbers of:
(1501 ± 4) cm⁻¹, (1472 ± 4) cm⁻¹ and (1400 ± 4) cm⁻¹; or
(1501 ± 4) cm⁻¹, (1472 ± 4) cm⁻¹, (1400 ± 4) cm⁻¹ and (1328 ± 4) cm⁻¹; or
(1645 ± 4) cm⁻¹, (1501 ± 4) cm⁻¹, (1472 ± 4) cm⁻¹, (1400 ± 4) cm⁻¹ and (1328 ± 4) cm⁻¹; or
(1645 ± 4) cm⁻¹, (1587 ± 4) cm⁻¹, (1501 ± 4) cm⁻¹, (1472 ± 4) cm⁻¹, (1400 ± 4) cm⁻¹ and (1328 ± 4) cm⁻¹; or
(1645 ± 4) cm⁻¹, (1587 ± 4) cm⁻¹, (1501 ± 4) cm⁻¹, (1472 ± 4) cm⁻¹, (1436 ± 4) cm⁻¹, (1400 ± 4) cm⁻¹ and (1328 ± 4) cm⁻¹; or
(1645 ± 4) cm⁻¹, (1587 ± 4) cm⁻¹, (1501 ± 4) cm⁻¹, (1472 ± 4) cm⁻¹, (1436 ± 4) cm⁻¹, (1400 ± 4) cm⁻¹, (1328 ± 4) cm⁻¹ and (1294 ± 4) cm⁻¹; or
(1645 ± 4) cm⁻¹, (1587 ± 4) cm⁻¹, (1501 ± 4) cm⁻¹, (1472 ± 4) cm⁻¹, (1436 ± 4) cm⁻¹, (1400 ± 4) cm⁻¹, (1328 ± 4) cm⁻¹, (1294 ± 4) cm⁻¹and (1211 ± 4) cm-¹; or
(1645 ± 4) cm⁻¹, (1587 ± 4) cm⁻¹, (1501 ± 4) cm⁻¹, (1472 ± 4) cm⁻¹, (1436 ± 4) cm⁻¹, (1400 ± 4) cm⁻¹, (1328 ± 4) cm⁻¹, (1294 ± 4) cm⁻¹, (1211 ± 4) cm⁻¹ and (681 ± 4) cm⁻¹,
when measured at a temperature in the range of from 20 to 30 °C and a wavelength of 785 nm.

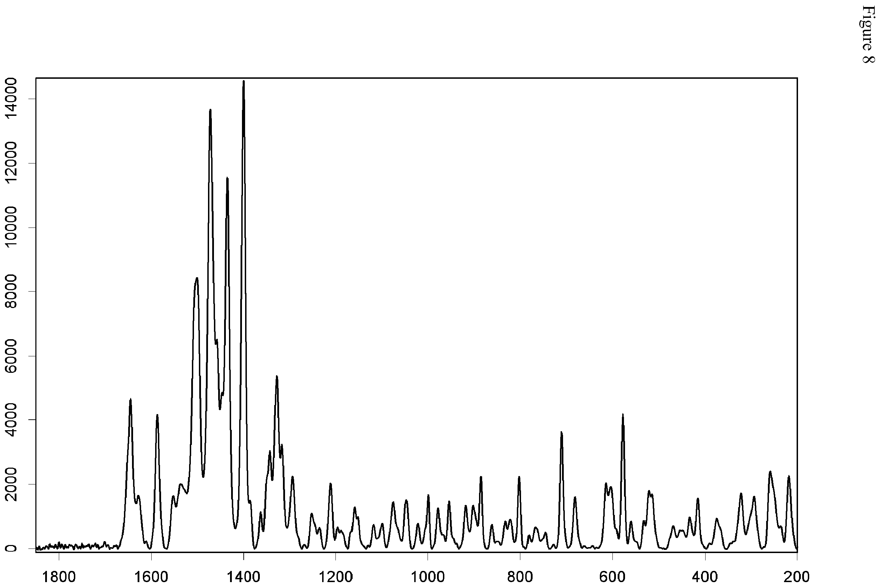

In yet another embodiment, the present invention relates to the 2,2,2-trifluoroethanol solvate of bictegravir sodium
characterized by having a Raman spectrum essentially the same as shown in Figure 8 of the present invention, when measured at a temperature in the range of from 20 to 30 °C and a wavelength of 785 nm.

The hereinabove described solvate preferably is a 2,2,2-trifluoroethanol monosolvate.

In another aspect, the present invention relates to a process for the preparation of crystalline Form II of bictegravir sodium of the present invention or the composition comprising Form II of bictegravir sodium as defined above comprising:
(i) providing a solution comprising bictegravir sodium and 2,2,2-trifluoroethanol;
(ii) crystallizing bictegravir sodium from the solution provided in step (i);
(iii)optionally separating at least a part of the crystals obtained in step (ii) from their mother liquor;
(iv)optionally washing the isolated crystals obtained in step (iii);
(v) drying the crystals obtained in any one of steps (ii) to (iv), wherein drying is performed under an atmosphere having a temperature in the range of from 50 °C to 150 °C and a vaccum of about 1 to 50 mbar;

Bictegravir may be prepared according to the teaching of WO 2014/100323 A1, in particular according to the teaching of Example 42 therein. The solution of step (i) of the above described procedure may be prepared by reacting Bictegravir in the presence of 2,2,2-trifluoroethanol with a basic sodium source, e.g. sodium hydroxide which is preferably applied as aqueous solution. Alternatively, the solution in step (i) of the above described process may be prepared starting from bictegravir sodium and dissolving bictegravir sodium in 2,2,2-trifluoroethanol. The bictegravir sodium starting material may be prepared by reacting bictegravir with aqueous sodium hydroxide solution in a suitable solvent such as water, ethanol, THF or any mixtures thereof. Bictegravir sodium may for example be prepared according to Reference Example 1 herein. WO 2015/196116 A1, in particular the specific example disclosed in the paragraph bridging pages 38 and 39, also provides a way of producing bictegravir sodium.

The solution of step (i) in the above describe process of the present invention may be prepared at room temperature or by heating bictegravir sodium in trifluoroethanol to a temperature in the range of from about 40 °C to reflux temperature for example to a temperature in the range of from about 50 to 70 °C. The bictegravir sodium concentration of said solution usually ranges from about 25 to 100 g/L, preferably from about 40 to 60 g/L and most preferably from about 50 to 55 g/L. Optionally, the solution may be filtered in order to remove any potentially undisolved particles.

After having obtained a clear solution comprising bictegravir sodium and 2,2,2-trifluoroethanol, bictegravir sodium is crystallized in the next step (ii). Crystallization is initiated by lowering the temperature of the solution provided in step (i) to a temperature in the range of from -25 to 30 °C, preferably from about -10 to 10 °C. Optionally, bictegravir sodium Form II seed crystals may be added in order to intiate crystallization and/or to control the particle size distribution of the finally obtained material. The seed crystal amount may range from about 1 to 10 weight% based on the amount of bictegravir sodium present in the solution. Once the material crystallized and the obtained suspension has reached the final target temperature, the obtained suspension is preferably further stirred at said temperature for a period which allows for plentiful crystallization and/or ripening of the crystals. Usually, the suspension is further stirred for a period ranging from about 1 to 48 hours, preferably from about 2 to 24 hours and most preferably from about 3 to 12 hours such as 4 to 6 hours.

Optionally, in the next step, at least a part of the crystals are separated from their mother liquor. Preferably, the crystals are separated from their mother liquor by any conventional method such as filtration, centrifugation, solvent evaporation or decantation, more preferably by filtration or centrifugation and most preferably by filtration.

In a further optional step, the isolated crystals may be washed with a suitable solvent, for example an organic solvent selected from 2,2,2-trifluoroethanol or ethanol.

The so obtained material was found to be a 2,2,2-trifluoromethanol solvate of bictegravir sodium, which can be desolvated by applying the conditions disclosed below.

The crystals obtained from any one of steps (ii) to (iv), preferably from step (iii) or (iv) are subsequently dried, wherein drying is performed at a temperature in the range of from about 50 to 150 °C for example at about 50 °C, 60 °C, 70 °C, 80 °C, 90 °C, 100 °C, 110 °C, 120 °C, 130 °C, 140 °C or 150 °C and a vaccum in the range of from about 1 to 50 mbar, for example in the range of from about 10 to 30 mbar. Typically, drying is performed for a period in the range of from about 6 to 72 hours, preferably from 10 to 48 hours, more preferably from 12 to 24 hours. Drying is performed, until most of the 2,2,2-trifluoroethanol solvate, preferably all of the 2,2,2-trifluoroethanol solvate obtained from any one of steps (ii) to (iv) of the above described process has desolvated to the desired bictegravir sodium Form II of the present invention. Said convertion can for example be detected by PXRD. One indication that the desolvation is complete and that drying can be terminated is the disappearance of the most intensive reflection at (5.8 ± 0.2) ° 2-Theta in the PXRD of the 2,2,2-trifluoroethanol solvate, which is not visible in the PXRD of phase pure bictegravir sodium Form II.

As already mentioned above bictegravir sodium Form II of the present invention combines the advantageous properties of high physical stability and high solubility and is therefore the preferred solid form of bictegravir sodium for the preparation of a pharmaceutical composition.

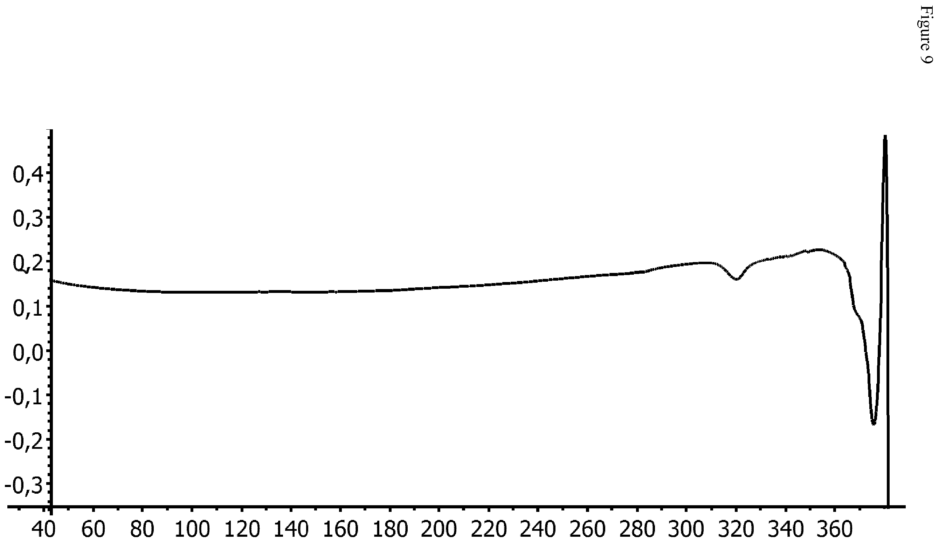

Despite being a metastable polymorph, it was found in a DSC experiment performed at a heating rate of 10 K/min that bictegravir sodium Form II of the present invention is extremely resistant against temperature stress and only changes its crystal structure, when heated to temperatures well above 300 °C (see experimental part and Figure 9 herein). Said solid-solid phase transformation was confirmed by hot stage microscopy and powder X-ray diffraction. E.g. a Form II sample which was heated just above the transition temperature and then cooled back to room temperature showed a PXRD in accordance with the PXRD of Form I of WO 2015/196116 A1. From said thermal behaviour it can be concluded, first of all that Form II is highly stable against temperature stress and only changes its crystal structure at temperatures which a pharmaceutical material will never experience during processing and storage and secondly, that Form I and Form II are monotropically related with Form I being the thermodynamically stable polymorph below its melting point (see Heat-of-Transition rule", A. Burger and R. Ramberger, "On the Polymorphism of Pharmaceuticals and Other Molecular Crystals. I" Microchim. Acta II, (1979) 259-271). The fact that Form II of bictegravir sodium of the present invention possesses a higher solubility compared to Form I of bictegravir sodium of WO 2015/196116 A1 is very much appreciated, since the higher solubility of Form II also translates to higher dissolution and most importantly higher bioavailability, which is of utter importance for a low soluble substance like bictegravir. Therefore, Form II of bictegrvir sodium of the present invention is the preferred form to be used in a pharmaceutical composition intended to be administered to a patient in need of such a therapy.

Hence, in a further aspect the present invention relates to the use of bictegravir sodium Form II of the present invention as defined above for the preparation of a pharmaceutical composition.

In a further aspect, the present invention relates to a pharmaceutical composition comprising bictegravir sodium Form II as defined above, preferably in an effective and/or predetermined amount, and at least one pharmaceutically acceptable excipient. Most preferably, the pharmaceutical composition of the present invention is an oral solid dosage form, such as a tablet or a capsule. Preferably, the pharmaceutical composition of the present invention is a tablet, most preferably a film-coated tablet. In one embodiment, the tablet is film-coated with a coating material containing polyvinyl alcohol (e.g. partially hydrolyzed), iron oxide, talc, and titanium dioxide.

The at least one pharmaceutically acceptable excipient, which is comprised in the pharmaceutical composition of the present invention, is preferably selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof. More preferably, the at least one pharmaceutically acceptable excipient is selected from the group consisting of fillers, diluents, lubricants, disintegrants and coating materials. In one embodiment all of these pharmaceutically acceptable excipients are comprised by the pharmaceutical composition of the present invention.

In a preferred embodiment, the at least one pharmaceutically acceptable excipient is selected from the group consisting of microcrystalline cellulose, croscarmellose sodium and magnesium stearate. In a preferred embodiment, all of these pharmaceutically acceptable excipients are comprised by the pharmaceutical composition of the present invention.

Preferably, the present invention relates to a pharmaceutical composition as described above, wherein the predetermined and/or effective amount of bictegravir sodium Form II is selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg calculated as bictegravir. Most preferably, the invention relates to a pharmaceutical composition as described above, wherein the predetermined and/or effective amount of bictegravir sodium Form II is 50 mg calculated as bictegravir.

In a further aspect, the present invention relates to a pharmaceutical composition comprising bictegravir sodium Form II as defined above, preferably in an effective and/or predetermined amount, and at least one pharmaceutically acceptable excipient and optionally one or more additional active pharmaceutical ingredient(s). Most preferably, the pharmaceutical composition of the present invention is an oral solid dosage form, such as a tablet or a capsule. Preferably, the pharmaceutical composition of the present invention is a tablet, most preferably a film-coated tablet. In one embodiment, the tablet is film-coated with a coating material containing polyvinyl alcohol (e.g. partially hydrolyzed), iron oxide, talc, and titanium dioxide.

The at least one pharmaceutically acceptable excipient, which is comprised in the pharmaceutical composition of the present invention, is preferably selected from the group consisting of carriers, fillers, diluents, lubricants, sweeteners, stabilizing agents, solubilizing agents, antioxidants and preservatives, flavouring agents, binders, colorants, osmotic agents, buffers, surfactants, disintegrants, granulating agents, coating materials and combinations thereof. More preferably, the at least one pharmaceutically acceptable excipient is selected from the group consisting of fillers, diluents, lubricants, disintegrants and coating materials. In one embodiment all of these pharmaceutically acceptable excipients are comprised by the pharmaceutical composition of the present invention.

In a preferred embodiment, the at least one pharmaceutically acceptable excipient is selected from the group consisting of microcrystalline cellulose, croscarmellose sodium and magnesium stearate. In a preferred embodiment, all of these pharmaceutically acceptable excipients are comprised by the pharmaceutical composition of the present invention.

Preferably, the present invention relates to a pharmaceutical composition as described above, wherein the predetermined and/or effective amount of bictegravir sodium Form II is selected from the group consisting of 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg and 100 mg calculated as bictegravir. Most preferably, the invention relates to a pharmaceutical composition as described above, wherein the predetermined and/or effective amount of bictegravir sodium Form II is 50 mg calculated as bictegravir.

In another preferred embodiment, the one or more additional active pharmaceutical ingredient(s) is/are selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

In a particular preferred embodiment, the one or more additional active pharmaceutical ingredient(s) is/are selected from the group consisting of emtricitabine or a pharmaceutically acceptable salt thereof and tenofovir alafenamide or a pharmaceutically acceptable salt thereof, most preferably the one or more additional active pharmaceutical ingredients are emtricitabine and tenofovir alafenamide hemifumarate.

In certain embodiments the pharmaceutical composition comprises 50 mg bictegravir sodium Form II calculated as bictegravir, 25 mg tenofovir alafenamide or a pharmaceutically acceptable salt thereof calculated as tenofovir alafenamide, and 200 mg emtricitabine or a pharmaceutically acceptable salt thereof calculated as emtricitabine. For instance, in certain embodiments, the pharmaceutical composition comprises 52.5 mg bictegravir sodium Form II, 28 mg tenofovir alafenamide hemifumarate and 200 mg emtricitabine. In a particular preferred embodiment, the pharmaceutical composition of the present invention is a bilayer tablet, comprising a first layer comprising bictegravir sodium Form II and a second layer comprising tenofovir alafenamide or a pharmaceutically acceptable salt thereof and emtricitabine or a pharmaceutically acceptable salt thereof. Preferably, the invention relates to a bilayer tablet, comprising a first layer comprising bictegravir sodium Form II and a second layer comprising tenofovir alafenamide hemifumarate and emtricitabine.

Preferably, the present invention relates to a pharmaceutical composition as described above, wherein the pharmaceutical composition is to be administered once-daily.

In a further aspect, the present invention relates to the pharmaceutical composition as described above for use as a medicament.

In yet another aspect, the present invention relates to the pharmaceutical composition as described above for use in the treatment or prophylaxis of viral infections caused by DNA viruses, RNA viruses, herpesviruses (e.g. CMV, HSV 1, HSV 2, VZV), retroviruses, hepadnaviruses (e.g. HBV), papillomavirus, hantavirus, adenoviruses and HIV.

In a particular embodiment, the present invention relates to the pharmaceutical composition as described above for use in the treatment and/or prophylaxis of HIV-1 infections.

Also disclosed is a method of treating or prophylactically preventing HIV-1 infections by administering the pharmaceutical composition as described above to a patient in need of such a treatment and/or prophylaxis.

In another aspect the present invention relates to the pharmaceutical composition as described above intended for use in the treatment of HIV-1 infections in combination with one or more additional active pharmaceutical ingredient(s) selected from the group consisting of entry/fusion inhibitors, reverse transcriptase inhibitors (RTIs), integrase strand transfer inhibitors (INSTI), maturation inhibitors, protease inhibitors (PIs) or mixtures thereof. In a further preferred embodiment, the entry/fusion inhibitors are selected from the group consisting of enfuvirtide, maraviroc, vicriviroc, cenicriviroc, ibalizumab and fostemsavir or mixtures thereof, the reverse transcriptase inhibitors (RTIs) are selected from the group consisting of abacavir, didanosine, emtricitabine, lamivudine, stavudine, zidovudine, amdoxovir, apricitabine, censavudine, elvucitabine, racivir, stampidine, zalcitabine, tenofovir disoproxil, tenofovir alafenamide, efavirenz, nevirapine, delavirdine, etravirine, rilpivirine, doravirine or mixtures thereof, the integrase strand transfer inhibitors (INSTI) are selected from the group consisting of dolutegravir, elvitegravir, raltegravir and bictegravir or mixtures thereof, the maturation inhibitor is bevirimat and the protease inhibitors (PIs) are selected from the group consisting of amprenavir, fosamprenavir, indinavir, lopinavir, nelfinavir, ritonavir, saquinavir, atazanavir, darunavir, tipranavir or mixtures thereof.

A treatment in combination with one or more additional active pharmaceutical ingredient(s) can mean the administration of a pharmaceutical dosage form comprising the bictegravir sodium form II of the present invention and the one or more additional active pharmaceutical ingredient(s) in the same dosage form, for example as a fixed-dose combination.

Alternatively, a treatment in combination with one or more additional active pharmaceutical ingredient(s) can mean the administration of separate pharmaceutical dosage forms, one comprising the bictegravir sodium form II of the present invention, and the other(s) comprising the one or more additional active pharmaceutical ingredient(s) in separate dosage form(s). Typically in such a combination treatment instructions are provided that the pharmaceutical dosage form comprising the bictegravir sodium form II of the present invention is to be administered in combination with said separate dosage form(s) for the effective treatment of a viral invention, such as HIV-1 infection.

### EXAMPLES

The following non-limiting examples are illustrative for the disclosure and are not to be construed as to be in any way limiting for the scope of the invention.

### Example 1: Preparation of bictegravir sodium Form II

Bictegravir (1.50 g, e.g. prepared according to Example 42 of WO 2014/100323 A1) was dissolved in trifluoroethanol (30 mL) upon heating to 65 °C. To the solution aqueous NaOH (1M, 3.6 mL) was dropwise added. Thereafter, the solution was cooled to 0 °C in 4 hours, whereupon crystallization was observed at a temperature of approximately 40 °C. The obtained suspension was further stirred at 0 °C for 3 hours, before the crystals were collected by filtration and dried under vacuum (30 mbar) at 50 °C for 15 hours to obtain crystalline Form II of bictegravir sodium (1.30 g).

### Powder X-ray diffraction

PXRD was performed with a PANalytical X'Pert PRO diffractometer equipped with a theta/theta coupled goniometer in transmission geometry, Cu-Kalpha₁,₂ radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of bictegravir sodium Form II that appears for example at 7.5° 2-Theta can appear in the range of from 7.3 to 7.7° 2-Theta, preferably in the range of from 7.4 to 7.6° 2-Theta on most X-ray diffractometers under standard conditions.

A representative diffractogram of bictegravir sodium Form II is displayed in Figure 1 herein. The corresponding reflection list is provided in Table 1 below.

**Table 1: PXRD reflections and corresponding relative intensities of bictegravir sodium Form II in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Relative intensity [%]** | **Reflection position [° 2-Theta]** | **Relative intensity [%]** |
|---|---|---|---|
| 6.5 | 14 | 18.8 | 30 |
| 7.5 | 100 | 19.4 | 13 |
| 13.0 | 14 | 20.9 | 43 |
| 14.6 | 9 | 23.5 | 8 |
| 15.4 | 2 | 24.2 | 14 |
| 16.3 | 4 | 24.7 | 11 |
| 16.8 | 8 | 26.5 | 18 |
| 18.1 | 10 | 29.6 | 6 |

### Fourier transform infrared spectroscopy

Bictegravir sodium Form II was investigated by FTIR spectroscopy. The FTIR spectrum was recorded (obtained) on a MKII Golden Gate^{™} Single Reflection Diamond ATR cell with a Bruker Tensor 27 FTIR spectrometer with 4 cm⁻¹ resolution at a temperature in the range of from 20 to 30 °C. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of ± 2 cm⁻¹. Thus, the infrared peak of bictegravir sodium form II that appears for example at 1624 cm⁻¹ can appear between 1622 and 1626 cm⁻¹ on most infrared spectrometers under standard conditions.

A representative FTIR spectrum of bictegravir sodium Form II according to the present invention is displayed in Figure 5 and the corresponding peak list is provided in Table 2 below.

**Table 2: FTIR peak list of bictegravir sodium Form II according to the present invention; a typical precision of the wavenumbers is in the range of ± 2 cm⁻¹.**

| **Wavenumber [cm⁻¹]** | **Wavenumber [cm⁻¹]** | **Wavenumber [cm⁻¹]** |
|---|---|---|
| 3055 | 1290 | 898 |
| 2964 | 1252 | 847 |
| 1647 | 1208 | 828 |
| 1614 | 1158 | 803 |
| 1525 | 1128 | 756 |
| 1435 | 1110 | 727 |
| 1404 | 1069 | 709 |
| 1362 | 998 | 677 |
| 1315 | 950 | 616 |

### Raman spectroscopy

Bictegravir sodium Form II was investigated by Raman spectroscopy. The Raman spectrum was recorded with a RamanRxn 1 Raman spectrometer and a PhAT probe with 6 mm spot size and 250 mm maximum focal length from Kaiser Optical Systems using a 785 nm Invictus laser with 400 mW power and a measurement time of 15 seconds. The spectrum was recorded from 1850 to 200 cm⁻¹ with 4 cm⁻¹ resolution at ambient conditions. A typical precision of the wavenumber values is in the range of about ± 4 cm⁻¹. Thus, a Raman peak that appears for example at 1641 cm⁻¹ can appear between 1637 and 1645 cm⁻¹ on most Raman spectrometers under standard conditions. A representative Raman spectrum of bictegravir sodium Form II is displayed in Figure 7. The displayed spectrum was baseline corrected with OPUS 7.0 (from Bruker Optik GmbH) using the concave rubberband method with 15 iterations and 64 baseline points. A corresponding peak list is provided in Table 3 below.

**Table 3: Raman peak list of bictegravir sodium Form II according to the present invention; a typical precision of the wavenumbers is in the range of from ± 4 cm⁻¹.**

| **Wavenumber [cm⁻¹]** | **Wavenumber [cm⁻¹]** | **Wavenumber [cm⁻¹]** |
|---|---|---|
| 1641 | 1160 | 709 |
| 1626 | 1150 | 680 |
| 1586 | 1074 | 615 |
| 1517 | 1047 | 577 |
| 1474 | 1020 | 524 |
| 1436 | 1001 | 514 |
| 1404 | 981 | 415 |
| 1384 | 952 | 328 |
| 1363 | 918 | 307 |
| 1328 | 887 | 292 |
| 1294 | 818 | 258 |
| 1254 | 803 | 216 |
| 1211 | 759 | |

### Differential scanning calorimetry

Bictegravir sodium Form II was investigated by DSC, which was performed on a Mettler Polymer DSC R instrument. The sample (3.77 mg) was heated in a 40 microliter aluminium pan with a pierced aluminium lid from 25 to 400 °C at a rate of 10 K/min. Nitrogen (purge rate 50 mL/min) was used as purge gas.

The DSC curve of Form II shows a small exothermic peak with an onset temperature of about 313 °C and a peak temperature of about 320 °C (see also Figure 9 herein). This peak is due to a solid-solid phase transition from Form II of the present invention to Form I of WO 2015/196116 A1. Form I then starts to decompose at a temperature of about 370 °C.

### Thermo gravimetric analysis

Bictegravir sodium Form II was investigated by TGA, which was performed on a Mettler TGA/DSC 1 instrument. The sample (11.64 mg) was heated in a 100 microliter aluminum pan closed with an aluminum lid. The lid was automatically pierced at the beginning of the measurement. The sample was heated from 25 to 300 °C at a rate of 10 K/min. Nitrogen (purge rate 50 mL/min) was used as purge gas.

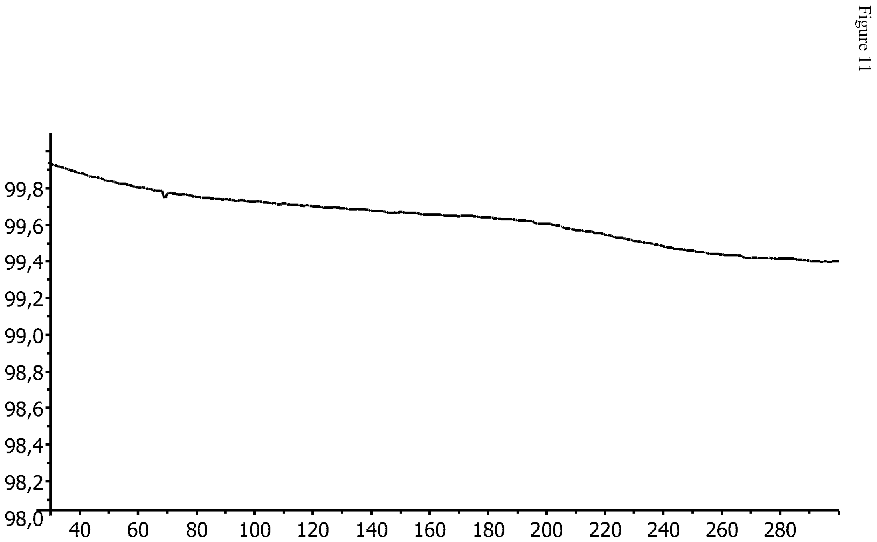

The TGA curve of Form II shows a mass loss of only about 0.5 weight% up to a temperature of about 300 °C (see Figure 11 herein). Hence, it can be concluded that neither water nor organic solvents are part of the crystal structure but the mass loss may rather be due to the release of residual solvent/water, which is loosely bound on the surface.

### Dissolution rate

The initial dissolution behavior of bictegravir sodium form II was compared with bictegravir sodium form I. Approximately 100mg Bictegravir-Na (Form I or Form II) were suspended at 24°C in 10ml solvent. Two different solvents were used for the study: 0,1M aqueous HCl solution and a phosphate buffer system (pH6,8; prepared according to "Küster Thiel - Rechentafeln dür die chemische Analytik").

After a stirring time of 1 minute a sample was taken and filtered throw a syringe filter. The received clear filtrate was analyzed by HPLC to determine the Bictegravir content. The HPLC-column was a YMC-Pack Pro C18 RS (150mm x 4.6mm) operated at 0.8 ml/min. Detection was at a wavelength of 254 nm. Mobile phases were sulfamic acid in water and acetonitrile, respectively, and were used as components of the gradient.

It was found that after 1 minute 0.186 mg/ml bictegravir sodium form II had dissolved in the pH6.8 buffer system, compared to only 0.059 mg/ml bictegravir sodium form I. In 0.1M HCl 0.220 mg/ml bictegravir sodium form II had dissolved, compared to only 0.085 mg/ml bictegravir sodium form I.

Thus bictegravir sodium form II is a storage stable (polymorphically stable as determined by XRPD for at least 1 month when stored at 40°C/75% rh) fast-dissolving crystalline form of bictegravir sodium with unexpected advantages over bictegravir sodium form I.

### Example 2: Preparation of bictegravir sodium trifluoroethanol solvate

Bictegravir (0.5 g, e.g. prepared according to Example 42 of WO 2014/100323 A1) was dissolved in trifluoroethanol (10 mL) upon heating to 70 °C. To the solution aqueous NaOH (1M, 1.22 mL) was added and the solution was further stirred at 65 °C for two hours. Thereafter, the solution was allowed to cool to room temperature, whereupon a suspension was obtained. The suspension was further stirred at room temperature for 5 hours before the crystals were collected by centrifugation in order to obtain bictegravir sodium trifluoroethanol solvate.

### Powder X-ray diffraction

PXRD was performed with a PANalytical X'Pert PRO diffractometer equipped with a theta/theta coupled goniometer in transmission geometry, Cu-Kalpha₁,₂ radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta. Thus, the diffraction peak of bictegravir sodium 2,2,2-trifluoroethanol solvate that appears for example at 5.8° 2-Theta can appear in the range of from 5.6 to 6.0° 2-Theta, preferably in the range of from 5.7 to 5.9 ° 2-Theta on most X-ray diffractometers under standard conditions.

A representative diffractogram of bictegravir sodium 2,2,2-trifluoroethanol solvate is displayed in Figure 2 herein. The corresponding reflection list is provided in Table 4 below.

**Table 4: PXRD reflections and corresponding relative intensities of bictegravir sodium 2,2,2-trifluoroethanol solvate in the range of from 2 to 30° 2-Theta; A typical precision of the 2-Theta values is in the range of ± 0.2° 2-Theta, preferably of ± 0.1° 2-Theta.**

| **Reflection position [° 2-Theta]** | **Relative intensity [%]** | **Reflection position [° 2-Theta]** | **Relative intensity [%]** |
|---|---|---|---|
| 5.8 | 100 | 21.0 | 44 |
| 9.1 | 3 | 21.4 | 3 |
| 11.6 | 1 | 22.3 | 7 |
| 12.5 | 3 | 23.5 | 14 |
| 15.9 | 10 | 23.7 | 9 |
| 16.8 | 39 | 24.1 | 2 |
| 17.5 | 23 | 24.6 | 3 |
| 18.1 | 4 | 25.0 | 14 |
| 18.8 | 8 | 27.8 | 4 |
| 19.5 | 31 | 28.2 | 9 |
| 20.0 | 3 | 29.6 | 5 |
| 20.4 | 6 | | |

### Fourier transform infrared spectroscopy

Bictegravir sodium 2,2,2-trifluoroethanol solvate was investigated by FTIR spectroscopy. The FTIR spectrum was recorded (obtained) on a MKII Golden Gate^{™} Single Reflection Diamond ATR cell with a Bruker Tensor 27 FTIR spectrometer with 4 cm⁻¹ resolution at a temperature in the range of from 20 to 30 °C. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of from about ± 2 cm⁻¹. Thus, the infrared peak of bictegravir sodium 2,2,2-trifluoroethanol solvate that appears for example at 1623 cm⁻¹ can appear between 1621 and 1625 cm⁻¹ on most infrared spectrometers under standard conditions.

A representative FTIR spectrum of bictegravir sodium 2,2,2-trifluoroethanol solvate according to the present invention is displayed in Figure 5 and the corresponding peak list is provided in Table 5 below.

**Table 5: FTIR peak list of bictegravir sodium 2,2,2-trifluoroethanol solvate according to the present invention; a typical precision of the wavenumbers is in the range of ± 2 cm⁻¹.**

| **Wavenumber [cm⁻¹]** | **Wavenumber [cm⁻¹]** | **Wavenumber [cm⁻¹]** |
|---|---|---|
| 3248 | 1313 | 900 |
| 3069 | 1290 | 832 |
| 2933 | 1251 | 802 |
| 1623 | 1207 | 767 |
| 1526 | 1157 | 747 |
| 1434 | 1106 | 711 |
| 1400 | 1073 | 679 |
| 1370 | 998 | 614 |
| 1344 | 952 | |

### Raman spectroscopy

Bictegravir sodium 2,2,2-trifluoroethanol solvate was investigated by Raman spectroscopy. The Raman spectrum was recorded with a RamanRxn 1 Raman spectrometer and a PhAT probe with 6 mm spot size and 250 mm maximum focal length from Kaiser Optical Systems using a 785 nm Invictus laser with 400 mW power and a measurement time of 15 seconds. The spectrum was recorded from 1850 to 200 cm⁻¹ with 4 cm⁻¹ resolution at ambient conditions. A typical precision of the wavenumber values is in the range of about ± 4 cm⁻¹. Thus, a Raman peak that appears for example at 1645 cm⁻¹ can appear between 1641 and 1649 cm⁻¹ on most Raman spectrometers under standard conditions. A representative Raman spectrum of bictegravir sodium 2,2,2-trifluoroethanol solvate is displayed in Figure 8. The displayed spectrum was baseline corrected with OPUS 7.0 (from Bruker Optik GmbH) using the concave rubberband method with 15 iterations and 64 baseline points. A corresponding peak list is provided in Table 6 below.

**Table 6: Raman peak list of bictegravir sodium 2,2,2-trifluoroethanol solvate according to the present invention; a typical precision of the wavenumbers is in the range of from ± 4 cm⁻¹.**

| **Wavenumber [cm⁻¹]** | **Wavenumber [cm⁻¹]** | **Wavenumber [cm⁻¹]** |
|---|---|---|
| 1645 | 1075 | 681 |
| 1587 | 1047 | 578 |
| 1501 | 1022 | 560 |
| 1472 | 999 | 521 |
| 1436 | 978 | 415 |
| 1400 | 955 | 375 |
| 1343 | 919 | 322 |
| 1328 | 902 | 293 |
| 1294 | 886 | 258 |
| 1252 | 862 | 218 |
| 1211 | 802 | |
| 1159 | 711 | |

### Differential scanning calorimetry

Bictegravir sodium 2,2,2-trifuoroethanol solvate was investigated by DSC, which was performed on a Mettler Polymer DSC R instrument. The sample (2.95 mg) was heated in a 40 microliter aluminium pan with a pierced aluminium lid from 25 to 400 °C at a rate of 10 K/min. Nitrogen (purge rate 50 mL/min) was used as purge gas.

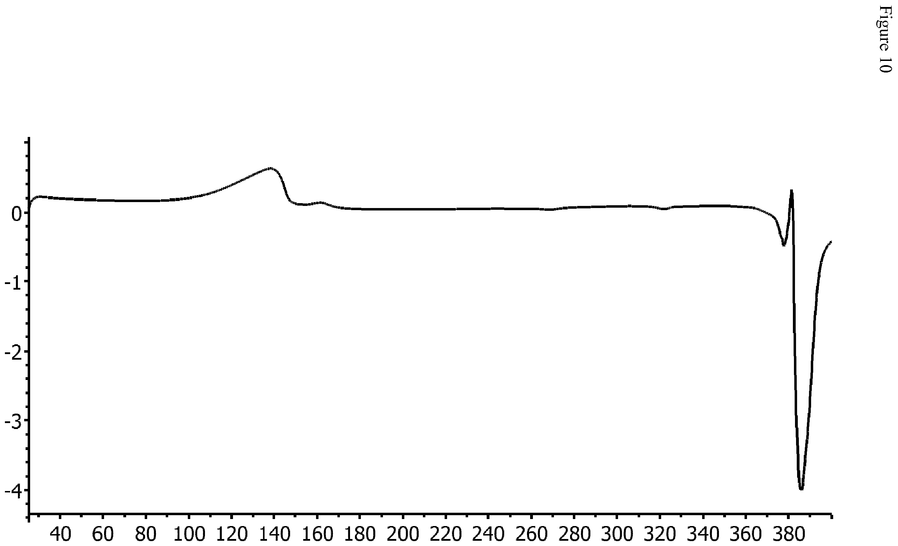

The DSC curve of the 2,2,2-trifluoroethanol solvate shows a broad endothermic peak with an onset temperature of about 104 °C and a peak temperature of about 138 °C followed by a smaller endothermic peak with an onset temperature of about 155 °C and a peak temperature of about 162 °C. These signals are due to the desolvation/dehydration process, which results in a transformation to Form II of the present invention. The remaining part of the DSC curve shows in essence the same behavior as described for Form II in Example 1 above, including the exothermic peak caused by the solid phase transformation to Form I with an onset temperature of about 315 °C and a peak temperature of about 322 °C (see Figure 10 herein).

### Thermo gravimetric analysis

Bictegravir sodium 2,2,2-trifluoroethanol solvate was investigated by TGA, which was performed on a Mettler TGA/DSC 1 instrument. The sample (9.57 mg) was heated in a 100 microliter aluminum pan closed with an aluminum lid. The lid was automatically pierced at the beginning of the measurement. The sample was heated from 25 to 300 °C at a rate of 10 K/min. Nitrogen (purge rate 50 mL/min) was used as purge gas.

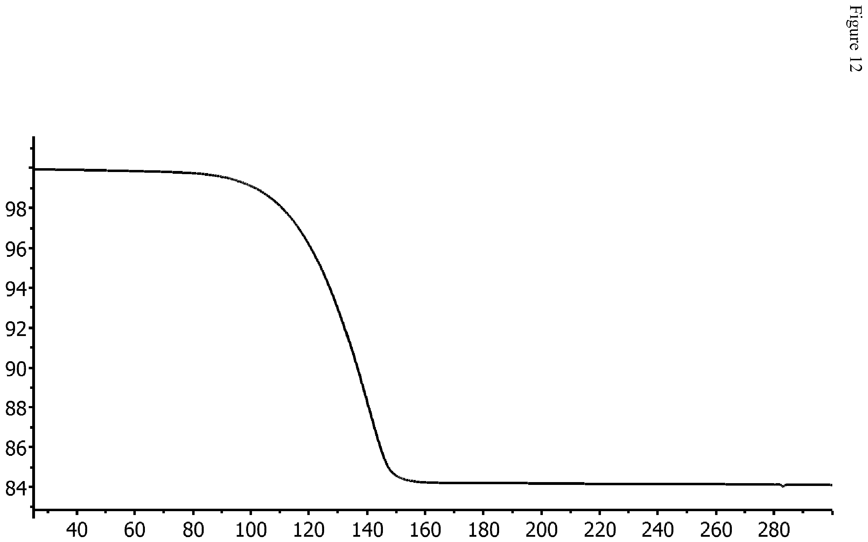

The TGA curve shows a mass loss of about 15.8 weight% up to a temperature of about 160 °C, which corresponds to approximately 0.9 mol 2,2,2-trifluoroethanol per mol bictegravir sodium (see Figure 12 herein).

### Example 3: Bilayer tablet comprising bictegravir sodium Form II/emtricitabine/tenofovir alafenamide hemifumarate

| **Ingredient** | **mg/tablet** | |
|---|---|---|
| | first layer | second layer |
| Bictegravir Na Form II | 52.5^{∗} | |
| Emtricitabine | | 200 |

| Tenofovir alafenamide hemifumarate | | 28^{∗∗} |
|---|---|---|
| Microcrystalline cellulose | 246.3 | 113.2 |
| Croscarmellose sodium | 19.4 | 30.2 |
| Magnesium stearate | 4.9 | 5.7 |
| Layer weight | 323 | 377 |
| **Tablet core weight** | 700 | |
| Opadry II | 21 | |
| **Film Coated Tablet** | 721.2 | |

| | | |
|---|---|---|
| ^{∗}Equivalent to 50 mg bictegravir ^{∗∗}Equivalent to 25 mg tenofovir alafenamide | | |

The manufacturing/packaging procedure for the bictegravir sodium Form II/emtricitabine/tenofovir alafenamide hemifumarate tablets is divided into five unit processes:
1. mixing bictegravir sodium Form II with intergranular excipients, roller compaction or slugging, milling, and blending with extragranular excipients to yield the final powder blend for bictegravir sodium Form II
2. mixing emtricitabine and tenofovir alafenamide hemifumarate with intergranular excipients, dry granulation, milling, and blending with extragranular excipients to yield emtricitabine/tenofovir alafenamide hemifumarate powder blend
3. tablet compression to yield bilayer tablet cores
4. tablet film-coating to yield film-coated tablets; and
5. packaging

The manufacturing process steps to produce the final drug product are detailed below.

Bictegravir sodium Form II final powder blend:
1. Weigh bictegravir sodium Form II and the excipients (microcrystalline cellulose and croscarmellose sodium). Correct the weight of Bictegravir sodium Form II based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.

Emtricitabine/tenofovir alafenamide hemifumarate final powder blend:
5. Weigh emtricitabine and tenofovir alafenamide hemifumarate and the excipients (microcrystalline cellulose and croscarmellose sodium). Adjust the weight of emtricitabine and tenofovir alafenamide hemifumarate based on their corresponding drug content factors, with a concomitant adjustment to the weight of microcrystalline cellulose.
6. Blend in emtricitabine and tenofovir alafenamide hemifumarate, microcrystalline cellulose and croscarmellose sodium to a tumble blender and blend.
7. Blend in intergranular portion of magnesium stearate to the tumble blender and blend.
8. Dry granulate the resulting blend using roller compactor or slug the resulting blend and mill.
9. Blend in the extragranular portion of magnesium stearate.

Tableting:
10. Compress the final powder blend of bictegravir sodium Form II as the first layer and the emtricitabine/tenofovir alafenamide hemifumarate final powder blend as the second layer, with an appropriate main compression force to achieve a target hardness of 17 kP (range: 14 to 20 kP). The final powder blend is compressed to a target layer weight of 323 mg using a target total tablet weight of 700 mg.

Film-coating:
11. Prepare a suspension of Opadry^{®} II. Film-coat the tablet cores to achieve the target tablet weight gain of 3% (range 2-4%). Dry film-coated tablets prior to cooling and discharge.

### Example 4: Film-coated tablet comprising bictegravir sodium Form II

| Ingredient | mg/tablet |
|---|---|
| Bictegravir Na Form II | 52.5^{∗} |
| Microcrystalline cellulose | 246.3 |
| Croscarmellose sodium | 19.4 |
| Magnesium stearate | 4.9 |
| **Tablet core weight** | 323 |
| Opadry II | 10 |
| **Film Coated Tablet** | 333 |

| | |
|---|---|
| ^{∗}Equivalent to 50 mg bictegravir | |

The manufacturing/packaging procedure for the bictegravir sodium Form II tablets is divided into four unit processes:
1. mixing bictegravir sodium Form II with intergranular excipients, roller compaction or slugging, milling, and blending with extragranular excipients to yield the final powder blend for bictegravir sodium Form II
2. tablet compression to yield tablet cores
3. tablet film-coating to yield film-coated tablets; and
4. packaging

The manufacturing process steps to produce the final drug product are detailed below. Bictegravir sodium Form II final powder blend:
1. Weigh bictegravir sodium Form II and the excipients (microcrystalline cellulose and croscarmellose sodium). Correct the weight of Bictegravir sodium Form II based on the drug content factor, with a concomitant reduction in the weight of microcrystalline cellulose.
2. Blend in intergranular portion of magnesium stearate to the tumble blender and blend.
3. Dry granulate the resulting blend using roller compactor or slug the resulting blend and mill.
4. Add extragranular microcrystalline cellulose and magnesium stearate and blend.

Tableting:
5. Compress the final powder blend of bictegravir sodium Form II with an appropriate main compression force to achieve a target hardness of 17 kP (range: 14 to 20 kP). The final powder blend is compressed to a target total tablet weight of 323 mg.

Film-coating:
6. Prepare a suspension of Opadry^{®} II. Film-coat the tablet cores to achieve the target tablet weight gain of 3% (range 2-4%). Dry film-coated tablets prior to cooling and discharge.

**Reference Example 1:** Preparation of bictegravir sodium Form I of WO 2015/196116 A1

Bictegravir (100 mg, e.g. prepared according to Example 42 of WO 2014/100323 A1) was dissolved in ethanol (3.9 mL) upon heating to 70 °C. To the solution aqueous NaOH (1M, 220 microL) was added and the solution was allowed to cool to room temperature, whereupon crystallization occurred. The obtained suspension was further stirred at room temperature for two hours. Thereafter, the suspension was kept at 2-8°C overnight before the crystals were collected by filtration. The wet product was washed with ethanol and dried under vacuum at room temperature for 2 hours in order to obtain bictegravir sodium (98 mg, Form I).

## Claims

1. A crystalline form of bictegravir sodium (Form II) **characterized by** having a powder X- ray diffractogram comprising reflections at 2-Theta angles of (6.5 ± 0.2)°, (7.5 ± 0.2)° and (18.8 ± 0.2)°, when measured at a temperature in the range of from 20 to 30 °C with Cu-Kalpha_{1,2} radiation having a wavelength of 0.15419 nm.

2. The crystalline form of claim 1 **characterized by** having a powder X-ray diffractogram comprising additional reflections at 2-Theta angles of (19.4 ± 0.2)° and (20.9 ± 0.2)°, when measured at a temperature in the range of from 20 to 30 °C with Cu-Kalpha_{1,2} radiation having a wavelength of 0.15419 nm.

3. The crystalline form of claim 1 or 2 **characterized by** having a powder X-ray diffractogram comprising no reflection in the range of (5.5 ± 0.2)° 2-Theta, when measured at a temperature in the range of from 20 to 30 °C with Cu-Kalpha_{1,2} radiation having a wavelength of 0.15419 nm.

4. The crystalline form according to any one of the preceding claims **characterized by** having a Fourier transform infrared spectrum comprising peaks at wavenumbers of (3055 ± 2) cm 1 , (2964 ± 2) cm⁻¹ and (1614 ± 2) cm⁻¹ , when measured at a temperature in the range of from 20 to 30 °C with a diamond attenuated total reflection cell.

5. The crystalline form according to any one of the preceding claims **characterized by** having a Raman spectrum comprising peaks at wavenumbers of (1641 ± 4) cm⁻¹ , (1436 ± 4) cm⁻¹ and (680 ± 4) cm⁻¹ , when measured at a temperature in the range of from 20 to 30 °C and a wavelength of 785 nm.

6. A composition comprising the crystalline form according to any one of the preceding claims and at most 5 weight% of any other physical form of bictegravir sodium, based on the weight of the composition.

7. The composition according to claim 6, wherein the other physical form of bictegravir sodium is Form I **characterized by** having a powder X-ray diffractogram comprising reflections at 2-Theta angles of (5.5 ± 0.3)°, (16.1 ± 0.3)°, (22.1 ± 0.3)°, (23.3 ± 0.3)° and (28.5 ± 0.3)°, when measured at a temperature in the range of from 20 to 30 °C with Cu- Kalpha_{1,2} radiation having a wavelength of 0.15419 nm.

8. The composition according to claim 6, **characterized by** having a powder X-ray diffractogram comprising no reflection in the range of (5.5 ± 0.2)° 2-Theta, when measured at a temperature in the range of from 20 to 30 °C with Cu- Kalpha_{1,2} radiation having a wavelength of 0.15419 nm.

9. Use of the crystalline form as defined in any one of claims 1 to 5 or the composition as defined in any one of claims 6 to 8 for the preparation of a pharmaceutical composition.

10. A pharmaceutical composition comprising the crystalline form as defined in any one of claims 1 to 5 or the composition as defined in any one of claims 6 to 8, and at least one pharmaceutically acceptable excipient.

11. The pharmaceutical composition of claim 10, further comprising one or more additional active pharmaceutical ingredient(s).

12. The pharmaceutical composition of claim 11, wherein the additional pharmaceutical ingredients are emtricitabine and tenofovir alafenamide hemifumarate.

13. The pharmaceutical composition of claims 10 to 12 for use in the treatment and/or prophylaxis of HIV-1 infections.

14. The 2,2,2-trifluoroethanol solvate of bictegravir sodium.

## Patentansprüche

1. Kristalline Form von Bictegravir-Natrium (Form II), **dadurch gekennzeichnet, dass** sie bei Messung bei einer Temperatur im Bereich von 20 bis 30 °C mit Cu-Kalpha_{1,2}-Strahlung mit einer Wellenlänge von 0,15419 nm ein Röntgenpulverdiffraktogramm mit Reflexen bei 2-Theta-Winkeln von (6,5 ± 0,2)°, (7,5 ± 0,2)° und (18,8 ± 0,2)° aufweist.

2. Kristalline Form nach Anspruch 1, **dadurch gekennzeichnet, dass** sie bei Messung bei einer Temperatur im Bereich von 20 bis 30 °C mit Cu-Kalpha_{1,2}-Strahlung mit einer Wellenlänge von 0,15419 nm ein Röntgenpulverdiffraktogramm mit zusätzlichen Reflexen bei 2-Theta-Winkeln von (19,4 ± 0,2)° und (20,9 ± 0,2)° aufweist.

3. Kristalline Form nach Anspruch 1 oder 2, **dadurch gekennzeichnet, dass** sie bei Messung bei einer Temperatur im Bereich von 20 bis 30 °C mit Cu-Kalpha_{1,2}-Strahlung mit einer Wellenlänge von 0,15419 nm ein Röntgenpulverdiffraktogramm ohne Reflex im Bereich von (5,5 ± 0,2)° 2-Theta aufweist.

4. Kristalline Form nach einem der vorhergehenden Ansprüche, **dadurch gekennzeichnet, dass** sie bei Messung bei einer Temperatur im Bereich von 20 bis 30 °C mit einer Diamantzelle mit abgeschwächter Totalreflexion ein Fourier-Transform-Infrarotspektrum mit Peaks bei Wellenzahlen von (3055 ± 2) cm⁻¹, (2964 ± 2) cm⁻¹ und (1614 ± 2) cm⁻¹ aufweist.

5. Kristalline Form nach einem der vorhergehenden Ansprüche, **dadurch gekennzeichnet, dass** sie bei Messung bei einer Temperatur im Bereich von 20 bis 30 °C und einer Wellenlänge von 785 nm ein Raman-Spektrum mit Peaks bei Wellenzahlen von (1641 ± 4) cm⁻¹, (1436 ± 4) cm⁻¹ und (680 ± 4) cm⁻¹ aufweist.

6. Zusammensetzung, umfassend die kristalline Form nach einem der vorhergehenden Ansprüche und höchstens 5 Gew.-% einer anderen physikalischen Form von Bictegravir-Natrium, bezogen auf das Gewicht der Zusammensetzung.

7. Zusammensetzung nach Anspruch 6, wobei es sich bei der anderen physikalischen Form von Bictegravir-Natrium um Form I handelt, die **dadurch gekennzeichnet ist, dass** sie bei Messung bei einer Temperatur im Bereich von 20 bis 30 °C mit Cu-Kalpha_{1,2}-Strahlung mit einer Wellenlänge von 0,15419 nm ein Röntgenpulverdiffraktogramm mit Reflexen bei 2-Theta-Winkeln von (5,5 ± 0,3)°, (16,1 ± 0,3)°, (22,1 ± 0,3)°, (23,3 ± 0,3)° und (28,5 ± 0,3)° aufweist.

8. Zusammensetzung nach Anspruch 6, **dadurch gekennzeichnet, dass** sie bei Messung bei einer Temperatur im Bereich von 20 bis 30 °C mit Cu-Kalpha_{1,2}-Strahlung mit einer Wellenlänge von 0,15419 nm ein Röntgenpulverdiffraktogramm ohne Reflex im Bereich von (5,5 ± 0,2)° 2-Theta aufweist.

9. Verwendung der kristallinen Form gemäß einem der Ansprüche 1 bis 5 oder der Zusammensetzung gemäß einem der Ansprüche 6 bis 8 zur Herstellung einer pharmazeutischen Zusammensetzung.

10. Pharmazeutische Zusammensetzung, umfassend die kristalline Form gemäß einem der Ansprüche 1 bis 5 oder die Zusammensetzung gemäß einem der Ansprüche 6 bis 8 und mindestens einen pharmazeutisch unbedenklichen Hilfsstoff.

11. Pharmazeutische Zusammensetzung nach Anspruch 10, ferner umfassend einen oder mehrere zusätzliche pharmazeutische Wirkstoffe.

12. Pharmazeutische Zusammensetzung nach Anspruch 11, wobei es sich bei den zusätzlichen pharmazeutischen Wirkstoffen um Emtricitabin und Tenofoviralafenamidhemifumarat handelt.

13. Pharmazeutische Zusammensetzung nach den Ansprüchen 10 bis 12 zur Verwendung bei der Behandlung und/oder Prophylaxe von HIV-1-Infektionen.

14. 2,2,2-Trifluorethanol-Solvat von Bictegravir-Natrium.

## Revendications

1. Forme cristalline de bictégravir sodium (Forme II) **caractérisée par le fait qu'**elle présente un diffractogramme des rayons X sur poudre comprenant des réflexions à des angles 2-thêta de (6,5 ± 0,2)°, (7,5 ± 0,2)° et (18,8 ± 0,2)°, lorsqu'il est mesuré à une température dans la plage allant de 20 à 30 °C avec un rayonnement Cu-Kalpha_{1,2} possédant une longueur d'onde de 0,15419 nm.

2. Forme cristalline selon la revendication 1 **caractérisée par le fait qu'**elle présente un diffractogramme des rayons X sur poudre comprenant des réflexions supplémentaires à des angles 2-thêta de (19,4 ± 0,2)°, et (20,9 ± 0,2)°, lorsqu'il est mesuré à une température dans la plage allant de 20 à 30 °C avec un rayonnement Cu-Kalpha_{1,2} possédant une longueur d'onde de 0,15419 nm.

3. Forme cristalline selon la revendication 1 ou 2 **caractérisée par le fait qu'**elle présente un diffractogramme des rayons X sur poudre ne comprenant pas de réflexion dans la plage de (5,5 ± 0,2)° 2-thêta, lorsqu'il est mesuré à une température dans la plage allant de 20 à 30 °C avec un rayonnement Cu-Kalpha_{1,2} possédant une longueur d'onde de 0,15419 nm.

4. Forme cristalline selon l'une quelconque des revendications précédentes **caractérisée par le fait qu'**elle présente un spectre infrarouge a transformée de Fourier comprenant des pics à des nombres d'onde de (3 055 ± 2) cm⁻¹, (2 964 ± 2) cm⁻¹ et (1 614 ± 2) cm⁻¹, lorsqu'il est mesuré à une température dans la plage allant de 20 à 30 °C avec une cellule à réflexion totale atténuée de diamant.

5. Forme cristalline selon l'une quelconque des revendications précédentes **caractérisée par le fait qu'**elle présente un spectre Raman comprenant des pics à des nombres d'onde de (1 641 ± 4)cm⁻¹, (1 436 ± 4)cm⁻¹ et (680 ± 4) cm⁻¹, lorsqu'il est mesuré à une température dans la plage allant de 20 à 30 °C et à une longueur d'onde de 785 nm.

6. Composition comprenant la forme cristalline selon l'une quelconque des revendications précédentes et au plus 5 % en poids d'une quelconque autre forme physique de bictégravir sodium, sur la base du poids de la composition.

7. Composition selon la revendication 6, l'autre forme physique de bictégravir sodium étant la Forme I **caractérisée par le fait qu'**elle présente un diffractogramme des rayons X sur poudre comprenant des réflexions à des angles 2-thêta de (5,5 ± 0,3)°, (16,1 ± 0,3)°, (22,1 ± 0,3)°, (23,3 ± 0,3)° et (28,5 ± 0,3)°, lorsqu'il est mesuré à une température dans la plage allant de 20 à 30 °C avec un rayonnement Cu-Kalpha_{1,2} possédant une longueur d'onde de 0,15419 nm.

8. Composition selon la revendication 6, **caractérisée par le fait qu'**elle présente un diffractogramme des rayons X sur poudre ne comprenant pas de réflexion dans la plage de (5,5 ± 0,2)° 2-thêta, lorsqu'il est mesuré à une température dans la plage allant de 20 à 30 °C avec un rayonnement Cu-Kalpha_{1,2} possédant une longueur d'onde de 0,15419 nm.

9. Utilisation de la forme cristalline telle que définie dans l'une quelconque des revendications 1 à 5 ou de la composition telle que définie dans l'une quelconque des revendications 6 à 8 pour la préparation d'une composition pharmaceutique.

10. Composition pharmaceutique comprenant la forme cristalline telle que définie dans l'une quelconque des revendications 1 à 5 ou la composition telle que définie dans l'une quelconque des revendications 6 à 8, et au moins un excipient pharmaceutiquement acceptable.

11. Composition pharmaceutique selon la revendication 10, comprenant en outre un ou plusieurs ingrédients pharmaceutiques actifs supplémentaires.

12. Composition pharmaceutique selon la revendication 11, les ingrédients pharmaceutiques supplémentaires étant l'emtricitabine et l'hémifumarate ténofovir alafénamide.

13. Composition pharmaceutique selon les revendications 10 à 12 pour une utilisation dans le traitement et/ou la prophylaxie d'infections par le VIH-1.

14. Solvate avec le 2,2,2-trifluoroéthanol de bictégravir sodium.
